# EUROPEAN PATENT SPECIFICATION

(11) **EP 2 544 607 B1**
(45) Date of publication and mention of the grant of the patent: **04.05.2016**
(21) Application number: 11707316.3
(22) Date of filing: 25.02.2011
(51) Int. Cl.: A61B 17/68, A61B 17/82, A61B 17/84

(54) **FRACTURE FIXATION DEVICE**
BRUCHFIXIERUNGSVORRICHTUNG
DISPOSITIF DE RÉPARATION D'UNE FRACTURE

(30) Priority: 03.11.2010 US 938902; 08.03.2010 US 719337
(43) Date of publication of application: 16.01.2013
(73) Proprietor: Biomet Sports Medicine, LLC, Warsaw, Indiana 46582 (US)
(72) Inventor: STONE, Kevin T., Winona Lake Indiana 46590 (US); BERELSMAN, Brian K., Warsaw Indiana 46580 (US); WAGNER, Zachary, Warsaw Indiana 46580 (US)
(74) Representative: Mays, Julie
(86) International application number: PCT/US2011/026349
(87) International publication number: WO 2011/112371

(56) References cited:
- WO-A1-95/03003
- US-A1- 2008 140 092
- US-A1- 2009 054 928
- US-A1- 2009 177 233
- US-A1- 2009 318 961

## Description

### FIELD

The present disclosure relates to devices for fracture fixation, and more particularly to holding bone fragments together to permit healing.

### BACKGROUND

The statements in this section merely provide background information related to the present disclosure and may not constitute prior art.

After trauma or surgical intervention, there may be a need to fix bone fragments together to immobilize the fragments and permit healing. Compressive force can be applied to the bone fragments by encircling the bone fragments or bridging the fragments together across a broken or otherwise compromised portion of the bone. The compressive forces should be applied such that upon ingrowth of new bone, the fragments will heal together and restore strength to the trauma or surgical intervention site.

Accordingly, there is a need for apparatus and methods to apply compressive force to a bone to affect healing. Further, there is a need for an apparatus and related methods which are easy to use intraoperatively to accommodate various bone sizes, shapes, or locations of fractures.

Document US 2009/0054928 A1 discloses a method and apparatus for coupling a soft tissue implant into a locking cavity formed within a bone. The apparatus includes a member to pull the soft tissue implant into a femoral tunnel.

Document US 20090318961 A1 discloses a method and apparatus for surgically repairing a tear in soft tissue. A plurality of collapsible tubes are positioned about the suture. The collapsible tubes are pushed through soft tissue and orthopedic mesh on opposite sides of a tear in soft tissue.

### SUMMARY

According to an aspect of the present disclosure, an apparatus for securing a fractured or sectioned sternum in a patient's body is provided as described in claim 1 of the appended claims.

Further areas of applicability will become apparent from the description provided herein. It should be understood that the description and specific examples are intended for purposes of illustration only and are not intended to limit the scope of the present disclosure.

### DRAWINGS

The drawings described herein are for illustration purposes only and are not intended to limit the scope of the present disclosure in any way.

Only the assemblies comprising an attachment member 80' like the one shown in figures 18, 18A, 20, 21, 31 and 32 fall within the scope of the claims.
Figure 1 depicts an assembly having a closed flexible member holder and an open flexible member holder according to the present teachings;
Figure 2 depicts a frame having two open flexible member holders and an adjustable flexible member construct according to the present teachings;
Figure 3 depicts an elongated frame according to the present teachings;
Figure 4 depicts an elongated frame having a plurality of flexible member holders according to the present teachings;
Figures 5A through 5C depict frames defining openings to receive fasteners according to the present teachings;
Figures 6A and 6B depict adjustable flexible member constructs according to the present teachings;
Figure 7 depicts an assembly compressing a fractured or weakened bone according to the present teachings;
Figure 8 depicts an assembly compressing a fractured or weakened bone according to the present teachings;
Figure 9 depicts an assembly compressing a fractured or weakened bone according to the present teachings;
Figures 10A and 10B depict a frame having a post extending therefrom according to the present teachings;
Figure 11 depicts a frame having a post extending therefrom used in an assembly according to the present teachings;
Figure 12 depicts a frame having a post extending therefrom where the frame defines a plurality of openings to receive at least one fastener according to the present teachings;
Figure 13 depicts a side view of a surgical method according to the present teachings;
Figure 14 depicts a top view of a surgical method according to the present teachings;
Figures 15A-15C depict side views of fixation of a fracture in surgical methods according to the present teachings;
Figures 16A and 16B depict a spinal repair using apparatus according to the present teachings;
Figure 17 depicts an adjustable flexible member construct according to the present teachings;
Figures 17A and 17B depict an exemplary method of assembling the adjustable flexible construct of Figure 17 according to the present teachings;
Figure 18 depicts an exemplary assembly configuration of the flexible member construct of Figure 17 having an attachment member and an insertion member according to the present teachings;
Figure 18A depicts a side view of the attachment member of Figure 18 according to the present teachings;
Figures 19, 19A and 19B depict alternative flexible member constructs according to the present teachings;
Figures 20 and 21 depict exemplary views of the adjustable flexible member construct of Figure 17 in a surgical procedure for sternal closure according to the present teachings;
Figures 22 - 25 depict views of exemplary alternative attachment members associated with one or more of the adjustable flexible member constructs according to the present teachings;
Figures 26 and 27 depict views of the attachment members of Figures 22-25 in exemplary configurations for use in a sternal closure procedure according to the present teachings;
Figure 28 depicts a view of an exemplary use of the flexible member construct of Figure 19A in a surgical method for sternal closure according to the present teachings;
Figure 29 depicts an exemplary alternative attachment member according to the present teachings;
Figure 30 depicts exemplary configurations of the attachment member of Figure 29 associated with various adjustable flexible member constructs according to the present teachings;
Figure 31 depicts an exemplary surgical method for sternal closure according to the present teachings; and
Figure 32 depicts an exemplary surgical method for sternal closure according to the present teachings.

### DETAILED DESCRIPTION

The following description is merely exemplary in nature and is not intended to limit the present disclosure, application, or uses. It should be understood that throughout the drawings, corresponding reference numerals indicate like or corresponding parts and features. While the present disclosure relates to fracture fixation, the apparatus and methods of the present teachings can be used with other orthopedic and non-orthopedic procedures as well.

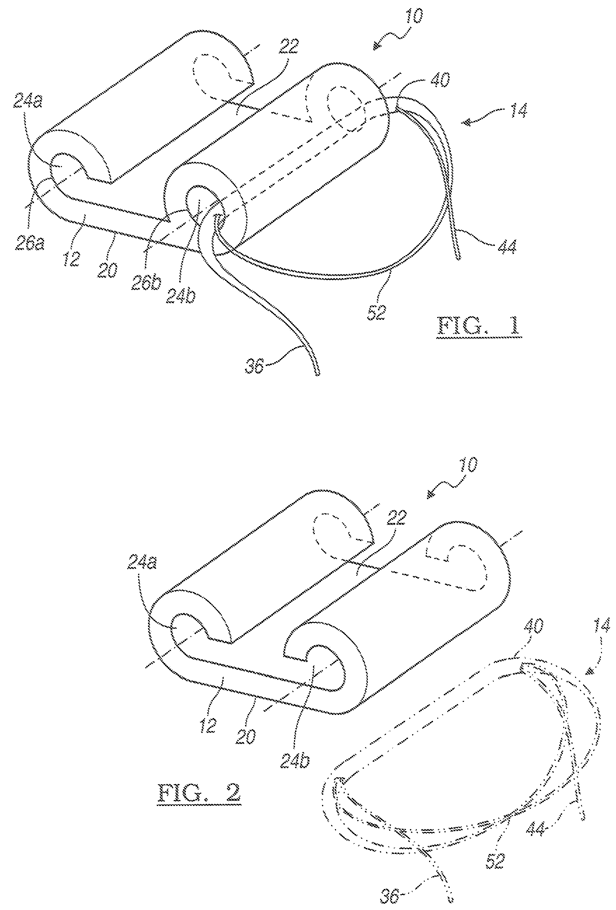

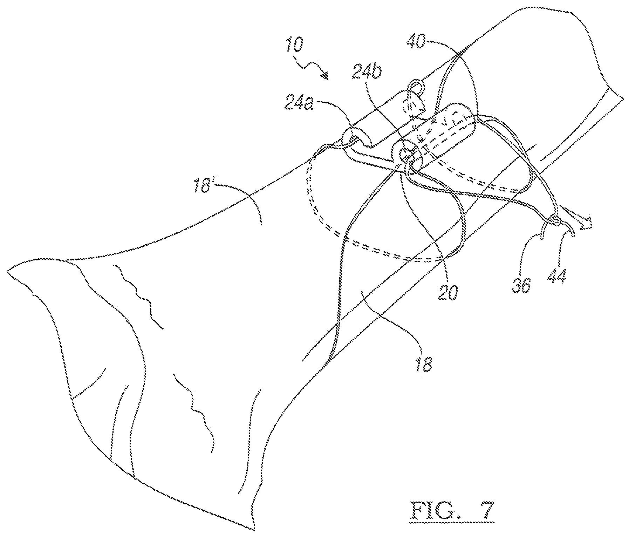

Referring to Figures 1 and 2, an assembly 10 is provided according to various embodiments of the present teachings. The assembly 10 includes a frame 12 and an adjustable flexible member construct 14. As further detailed below and as depicted in Figure 7, regions of the adjustable flexible member construct 14 are partially disposed in the frame 12 such that the adjustable flexible member construct 14 and the frame encircle a bone 16 having fragments 18 and 18' due to surgical intervention, injury, or disease. While the present disclosure may exemplify a fractured bone, it is understood that any of the reasons for bone compromise may be used with the present teachings. It is further understood that the frame 12 can be used in connection with other frames that are placed on a different or opposing face of the bone. The various embodiments disclosed herein can also be used to stabilize other implants, such as those used in revision surgery or for oncological purposes.

In various embodiments, the assembly 10, assembly 110 as detailed later herein, or the adjustable flexible member construct 14 alone is used to compresses the respective fragments together and to affect healing at the compromised bone 16. Bones suitable for use with the present teachings include any bone in the body, such as the vertebrae, long bones of the arms, legs or fingers; curved bones, such as the ribs; flat bones, such as those of the wrist or feet, for example, and the like. Any bones of the legs, arms, torso, hands, feet, head, are suitable for use with the apparatus and methods of the present teachings.

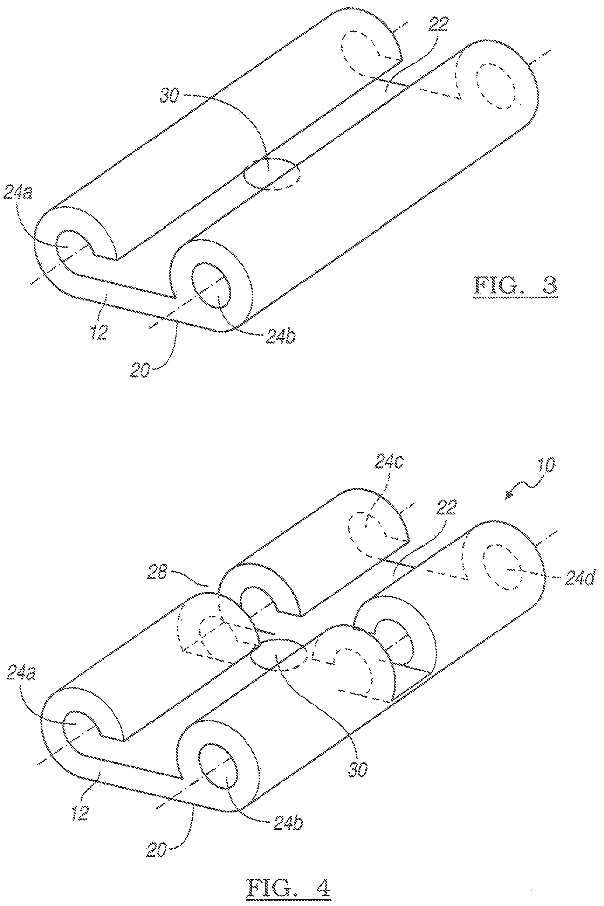

Referring to Figures 1 through 5C, the frame 12 includes a lower surface 20, an upper surface 22, and at least flexible member holders 24A and 24B defined by projections on the upper surface 22. In various embodiments, the frame 12 can be a one-piece, integral, monolithic structure. In various embodiments, the frame 12 can be made of a generally rigid material. The frame 12 can be made of a plastic or polymeric material, a metal, or a composite thereof. The frame 12 can be generally rectangular or square, or the frame 12 can be a rounded shape or a site-specific shape. For example, the lower surface 20 can be curved to conform to the desired bone. The frame 12 can be of a sufficient length to span across a region of both fragments 18 and 18'. The frame 12 can also span the entirety of the fractured area of the bone 16 and cover healthy adjacent bone 16, or the frame can be smaller than the fractured area of the bone 16. The frame 12 can also be elongated such that it spans beyond the length of the fracture or weakened area, such as the frames 12 shown in Figures 3 and 4.

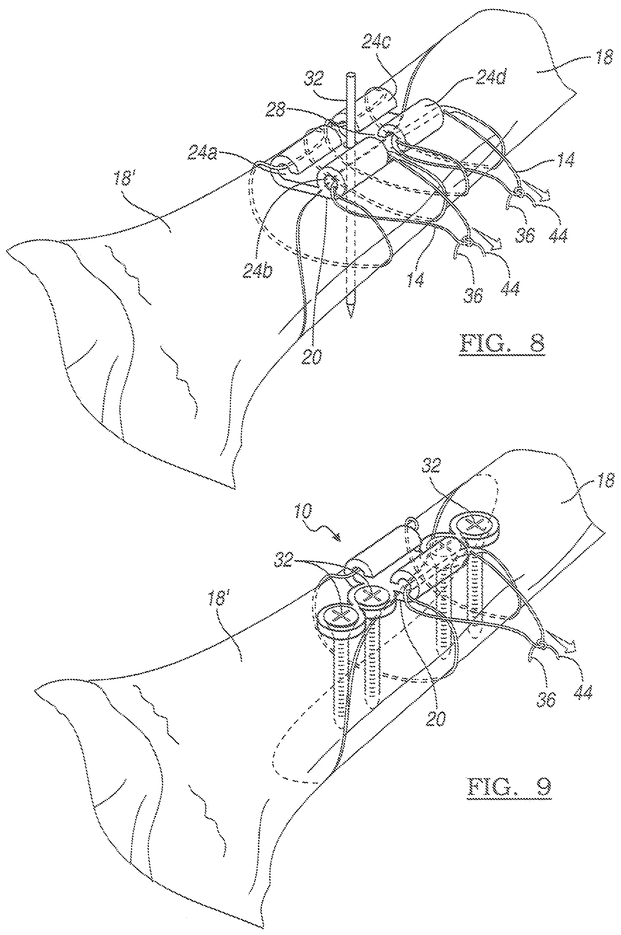

The frame lower surface 20 can include a flat surface or the lower surface 20 can be curved to accommodate the contour of the bone 16. The upper surface 22 of the frame 12 partially defines the openings 26A and 26B for the flexible member holders 24A and 24B, respectively. The flexible member holders 24A and 24B can be channels, a post, a pin, a hole, or other means by which to retain a flexible member on the frame 12. It is understood that the flexible member holders 24A and 24B need not be formed on the upper surface 22 and that the flexible member holders 24A and 24B can extend from the lower surface 20 and around the upper surface 22. As shown in Figures 4 and 8, the frame 12 can include a plurality of flexible member holders 24A-24D which can be separated by a space 28 between the sets of flexible member holders 24C and 24A, and 24D and 24B. Although various embodiments disclosed may relate to only two flexible member holders, it is understood that the processes of use are generally the same for assemblies having 2 through 8, or more flexible member holders.

Any of the flexible member holders 24A-24D can be open such that the adjustable flexible member construct 14 can be repeatedly manually placed and removed, or the flexible member holders 24A-24D can be closed such that the adjustable flexible member construct 14 is permanently housed therein and cannot be inadvertently removed without disassembling the adjustable flexible member construct 14. The flexible member holders 24A-24D can be sized to allow the adjustable flexible member construct 14 to freely slide therein. The flexible member holders 24A-24D can be pre-formed to be closed or can be initially provided as an open and subsequently crimped or pinched closed. Figure 1 depicts the frame having an open flexible member holder 24A and a closed flexible member holder 24B while Figure 2 depicts two open flexible member holders 24A and 24B.

In various embodiments, the flexible member holders 24A and 24B face each other or are opposed. This allows for the adjustable flexible member construct 14 to be disposed in a first flexible member holder, for example flexible member holder 24B, wrapped around the bone 16, and then be disposed in the other flexible member holder, for example flexible member holder 24A, and tightened when the flexible member construct 14 is engaged such that opposing force is applied to the opposing flexible member holder to provide compression.

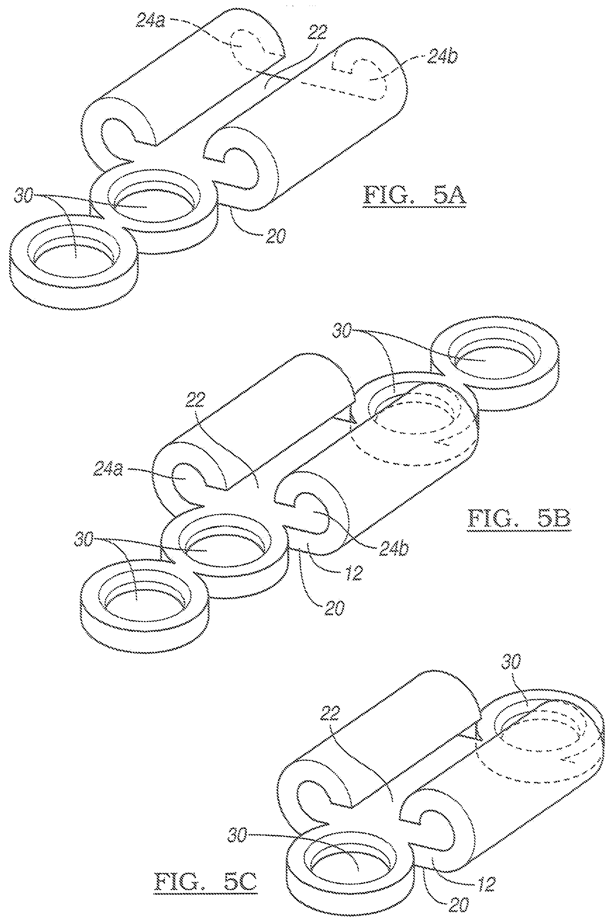

As shown in Figures 3 through 5C and Figures 8 and 9, the frame 12 can define at least one opening 30 in which to attach a fastener 32 such as a pin, screw, spike, or a combination or variation thereof to bone. The frame 12 can include a plurality of fastener openings 30 to accommodate multiples of the same or different fasteners 32. The fastener openings 30 can be placed along the periphery of the frame 12 as shown in Figures 5A through 5C, or the fastener openings 30 can cut through the lower surface 20 and upper surface 22 of the frame 12. The fastener openings 30 can be evenly placed on or about the frame 12, as shown in Figure 5B and Figure 9, or they can be asymmetrically placed on or about the frame 12, as shown in Figure 5A. It is understood that the fastener openings 30 can be placed anywhere along the frame 12 at any angle and can be placed within the interior of the frame 12.

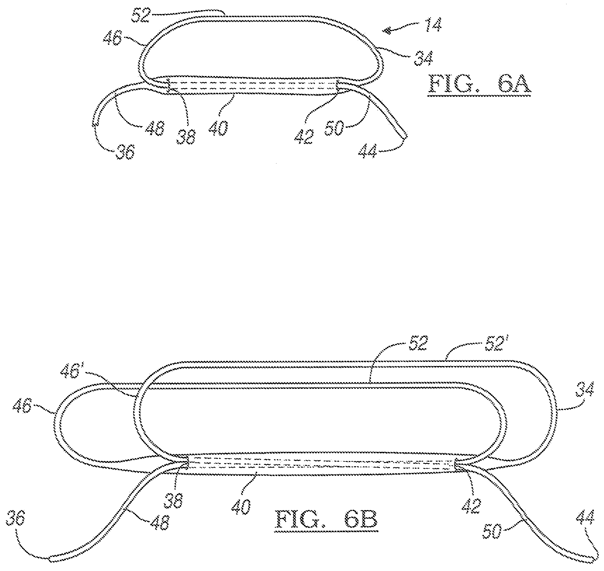

Referring to Figure 1, the frame 12 can be used to hold the adjustable flexible member construct 14 as depicted in Figures 6A and 6B. The adjustable flexible member construct 14 is fashioned from a flexible member 34 made of any biocompatible material that is flexible and can fold around and secure a bone 16. Exemplary materials include, but are not limited to, non-resorbable polymers, such as polyethylene or polyester, resorbable polymers, metals, and various combinations thereof. The materials can include those formed into a monofilament, multiple filaments, cables, and the like. In various embodiments, the adjustable flexible member construct 14 is made of a hollow material to allow for the appropriate folding and tensioning. In various embodiments, the adjustable flexible member construct 14 can be a suture. In such embodiments, the suture can be hollow or a braided or multiple-filament suture structure. In various embodiments, the adjustable flexible member construct 14 can define a substantially tubular hollow shape.

To form the adjustable flexible member construct 14, a first end 36 of the flexible member is passed through the first aperture 38 and through longitudinal passage 40 and out a second aperture 42. The second end 44 is passed through the second aperture 42, through the longitudinal passage 40 and out the first aperture 38. In various embodiments, the first and second apertures 38 and 42 are formed during the braiding process as loose portions between pairs of fibers defining the flexible member 34. Passing the ends 36 and 44 through the apertures 38 and 42 forms loops 46 and 46'. The longitudinal and parallel placement and advancement of the first and second ends 36 and 44 of the flexible member 34 within the longitudinal passage 40 resists the reverse relative movement of the first and second portions 48 and 50 of the flexible member 34 once it is tightened. A further discussion of the flexible member construct is provided in U.S. Patent Serial No. 11/541,506 filed on September 29, 2006 entitled "Method And Apparatus For Forming A Self-Locking Adjustable Suture Loop" assigned to Biomet Sports Medicine, Inc.

The loops 46 and 46' define mounts or summits 52 and 52' of the adjustable flexible member construct 14 and are disposed opposite from the longitudinal passage 40 such that when the summit 52 is disposed in a first flexible member holder 24A and the longitudinal passage 40 is disposed in a second flexible member holder 24B, the summit 52 and the longitudinal passage 40 remain stationary with respect to the frame 12, while the overall diameter of the adjustable flexible member construct 14 is decreased to compress the bone fragments 18 and 18'.

The tensioning of the ends 36 and 44 cause relative translation of the sides of the flexible member 34 with respect to each other. Upon applying tension to the first and second ends 36 and 44 of the flexible member 34, the size of the loop(s) 46 is reduced to a desired size or load. The flexible member 34 locks without knots due to the tensioning placed on the first and second ends 36 and 44. At this point, additional tension causes the body of the flexible member defining the longitudinal passage 40 to constrict about the portions 48 and 50 of the flexible members within the longitudinal passage 40. This constriction reduces the diameter of the longitudinal passage 40, thus forming a mechanical interface between the exterior surfaces of the first and second portions 48 and 50, as well as the interior surface of the longitudinal passage 40. This constriction causes the adjustable flexible member to "automatically" lock in a reduced or smaller diameter position.

In use, the assembly 10 is formed by coupling the adjustable flexible member construct 14 to the frame 12. The lower surface 20 is placed such that it abuts the bone fragments 18 and 18'. In embodiments where at least one flexible member holder 24A or 24B is closed, the summit 52 is placed in the open flexible member holder 24B opposite the longitudinal passage 40 disposed in the opposing closed flexible member holder 24A. The flexible member free ends 36 and 44 are engaged and pulled in the direction of the arrow shown in Figure 7 such that the diameter of the loop 46 is reduced and the bone fragments 18 and 18' are compressed. In embodiments where the longitudinal passage 40 is not pre-disposed in the closed flexible member holder 24A or where both flexible member holders are open, the longitudinal passage 40 and the summit 52 are placed in the respective, opposing flexible member holder and then the free ends 36 and 44 are engaged to tighten the adjustable flexible member construct 14 and secure the bone fragments 18 and 18'. No additional steps, such as knot tying, are required to secure the adjustable flexible member due to the automatic locking feature.

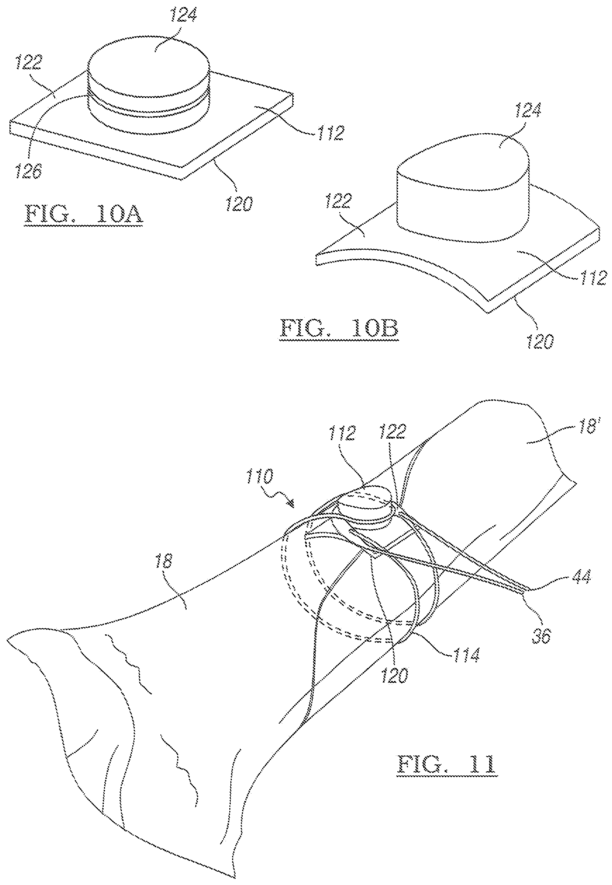

Turning to Figures 10A through 15C, in still other embodiments, an assembly 110 is provided. The assembly 110 shares several similarities with the assembly 10 detailed above. It is understood that the assembly 110 and the assembly 10 can have interchangeable features and the discussion of separate features on the respective assemblies is not intended to be a limitation of the present teachings.

The assembly 110 includes a frame 112 and an adjustable flexible member construct 114. The frame 112 includes a lower surface 120, an upper surface 122, and at least one flexible member holder, depicted as a post 124, thereon about which the adjustable flexible member construct 114 can be secured.

The post 124 sits proud to the upper surface 122 of the frame. The post 124 can be centered on the frame 112, or the post 124 can be placed at an off-center point on the frame 112. The post 124 can be generally smooth and cylindrical as shown in Figures 10A and 10B, or the post 124 can be a squared or have any other suitable geometry. The post 124 can include surface features by which the adjustable flexible member construct 114 can be disposed in or through, such as a notch, under cut, groove, or throughbore. An exemplary under cut 126 is shown in Figure 10A.

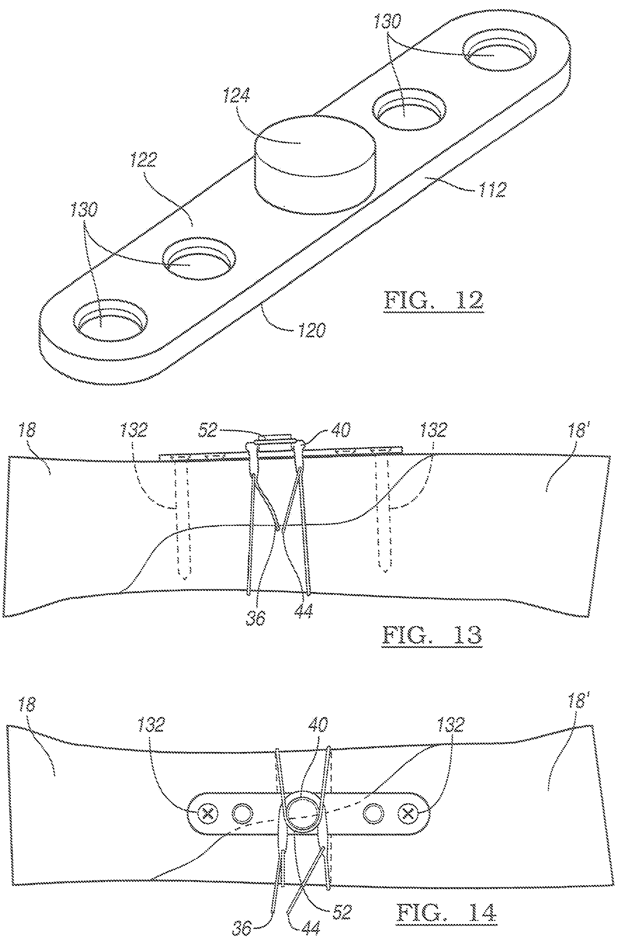

The frame 112 can have a flat profile or the frame 112 can have a slightly curved profile, such as those shown in Figures 10A and 10B, respectively. As shown in Figure 12, for example, the frame 112 can define openings 130 to receive fasteners 132 such as those detailed earlier herein. Although a plurality of evenly spaced fastener openings 130 are depicted on the frame 112 in Figure 12, it is understood that the fastener openings 130 can be placed anywhere along the periphery of the frame 112, can be placed through the center of the post 124, or can be asymmetrically placed. The fastener openings 130 can also be threaded to receive screws. In various embodiments, the fastener openings 130 can include both machine threads and bone engaging threads.

In use, the lower surface of the frame 112 is placed against the bone fragments 18 and 18'. Either of the summit 52 or the longitudinal passage 40 of the adjustable flexible member construct 14 is disposed about the post 124. The other of the summit 52 or the longitudinal passage 40 is partially circled about the bone 16 and is disposed on the opposite side of the post 124. In embodiments where the post 124 includes a notch 126, the adjustable flexible member construct 14 can be disposed in the notch 126 during the wrapping process. The ends 36 and 44 of the adjustable flexible member construct 14 are engaged to reduce the diameter of the adjustable flexible member construct 14 about the bone 16 and thereby compress the bone fragments 18 and 18'.

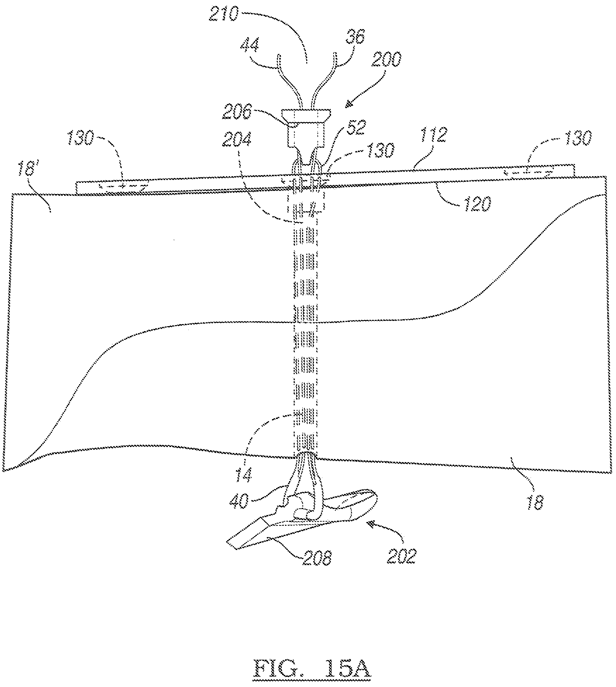

Turning to Figures 15A-15C, in various embodiments, the assembly 210 can include an upper fixation element 200 and a lower fixation element 202 having the adjustable flexible member construct spanning therebetween through an opening 204 formed in the bone fragments 18 and 18'. The upper fixation element 200 and lower fixation element 202 can independently be selected from a grommet 206, a toggle 208, a button 210, a screw tip 212, a screw head 214, or other similar items.

As shown in Figure 15A, the upper fixation element 200 is a grommet 206 and the lower fixation element is a toggle 208. The toggle 208 is used to hold the longitudinal passage 40 of the adjustable flexible member construct 14 and the opposing region is contained by the grommet 206 in bone fragment 18. The adjusting arms 36 and 44 are also passed through the grommet 206 and can be pulled to tighten the adjustable flexible member construct 14 and compress the bone fragments 18 and 18' together. The upper fixation element 200 and lower fixation element 202 are shown in connection with a fixation plate 112 where the grommet 206 is disposed in the opening 130 in the fixation plate 112. It is understood that the plate 112 can be used with either of the upper fixation element 200 or the lower fixation element 202.

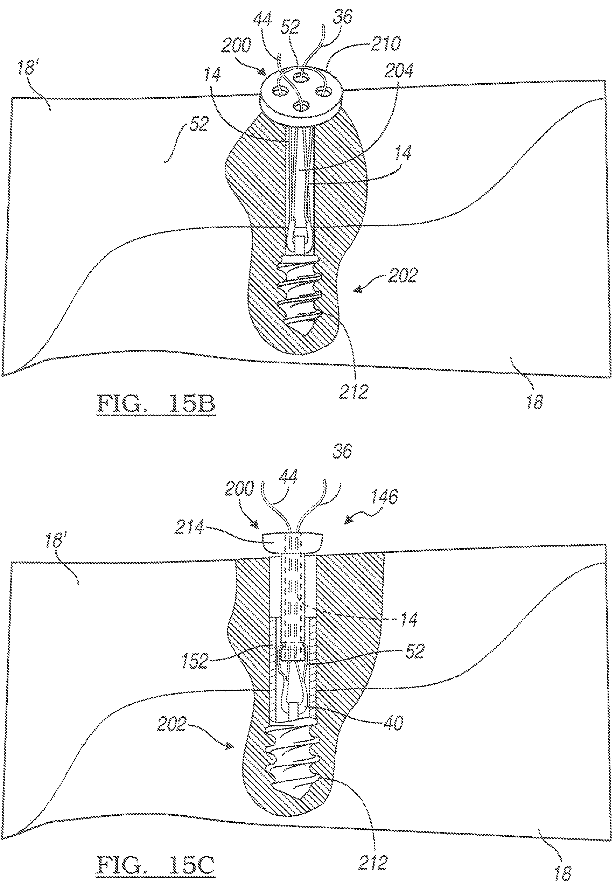

Figures 15B and 15C depict assemblies without the fixation plate 112 shown in Figure 15A. Similar to the previously discussed embodiment, an opening 204 is prepared in the bone fragments 18 and 18'. The opening 204 is a partial opening and does not extend all of the way through both bone fragments 18 and 18'. With specific reference to Figure 15B, a button 210 is used as the upper fixation element 200, and a screw tip 212 serves as the lower fixation element 202. The screw tip 212 retains the longitudinal passage 40 of the adjustable flexible member construct 14 and is fixed into the bone fragment 18. At least the free ends 36 and 44 and the summit 52 of the adjustable flexible member construct 14 are disposed through the button 210, through lacing for example, and can be adjusted to provide the secured fit and bone fragment 18 and 18' fixation.

Turning to Figure 15C, the upper fixation element 200 is depicted as a telescoping screw head 214, and the lower fixation element 202 is a screw tip 212. The screw tip 212 is fixed in the bone fragment 18 and retains the longitudinal passage 40 of the adjustable flexible member construct 14. The telescoping screw head 214 is used to retain the summit 52 and the adjustable free ends 36 and 44 of the adjustable flexible member construct 14 and can be compressed into the distally placed screw tip 212 to secure the fracture.

The present teachings further provide methods for securing a fractured or weakened bone 16 within a patient's body. The frame 12 is abutted against the fractured or weakened bone 16 such that the lower surface 20 sits against the bone. The frame 12 can be positioned to span across both sides of the fracture. The summit 52 or the longitudinal passage 40 is disposed in one of the flexible member holders 24A or 24B defined by the frame 12. The fractured or weakened bone 16 is then encircled by partially wrapping the adjustable flexible member construct 14 at least partially contained in the frame about the bone. The other of the summit 52 or the longitudinal passage 40 is fixed in the second, opposing flexible member holder 24B. In embodiments where a plurality of frames 12 or a plurality of flexible member holders 24A-24D are provided on a frame 12, the process can be repeated by employing several adjustable flexible member constructs 14 in various sets of flexible member holders, for example the set 24A/24C and the set 24B/24D of Figure 4 and Figure 8. The process can also be repeated by wrapping a single adjustable flexible member construct 14 about the area to engage several flexible member holders on the different frames, such as where the frames are used in tandem across a fractured or weakened bone.

The ends 36 and 44 of the adjustable flexible member construct 14 are engaged or pulled to reduce the size of the loop 46 and to cause the summit 52 and the longitudinal passage to press against the respective opposed flexible member holders. This compresses the bone fragments 18 and 18' at the compromised site. In embodiments where the frame 12 is made of a rigid material, engaging the free ends 36 and 44 does not cause the frame 12 to stretch, lengthen, or otherwise increase in size, thereby allowing for tighter compression. In embodiments utilizing fasteners 32 or 132, the fasteners can be secured to the bone fragments, 18 and 18' before or after the adjustable flexible member construct 14 is reduced about the bone 16. The flexible member constructs allow additional tensioning of each individual flexible member construct independently, so as to avoid any laxity that may occur to a flexible member construct as others are tightened.

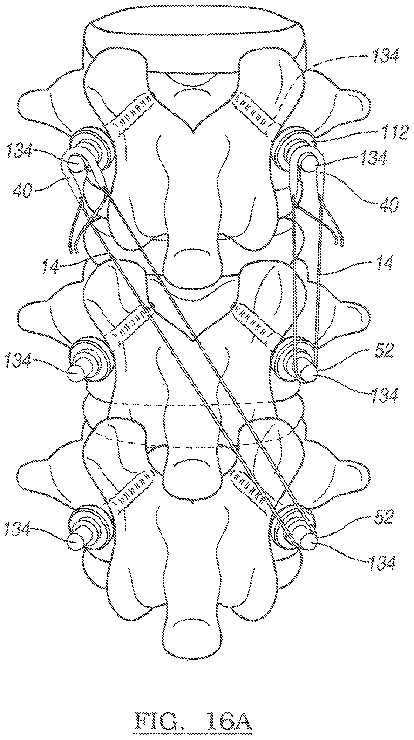

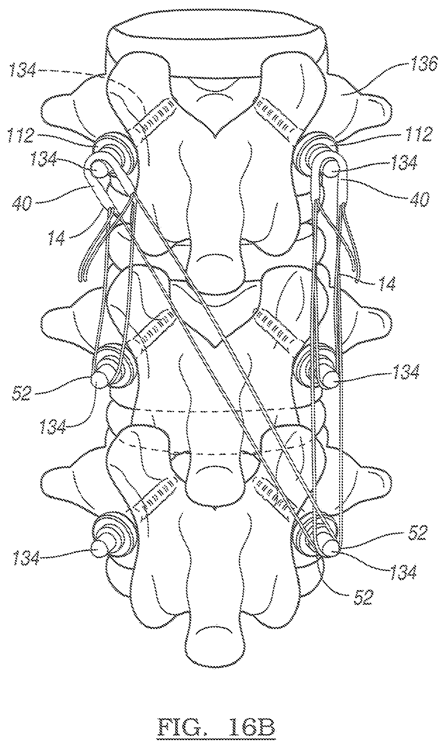

In still further embodiments such as those shown in Figures 16A and 16B, the frame 112 can be attached via a pedicle screw 134 which is affixed to vertebra 136. The pedicle screw 134 is passed through the fastener opening 130 which is defined by the post 124. The pedicle screws 134 can be linked together using the adjustable flexible member construct 14. As shown in Figure 16B, a single adjustable flexible member construct 14 can be attached to two or more assemblies 10. Alternatively, as shown in Figures 16A and 16B, multiple adjustable flexible member constructs 14 can be attached to one or more pedicle screws 134.

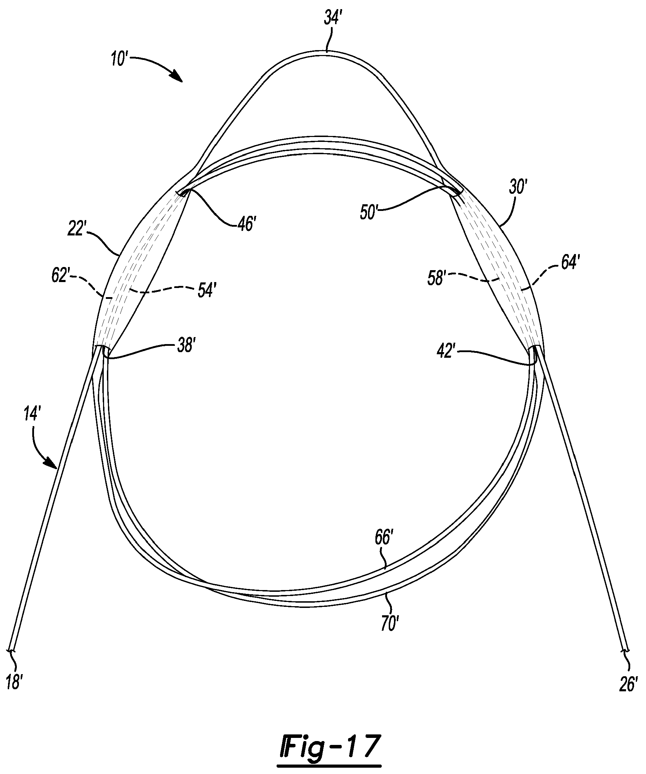

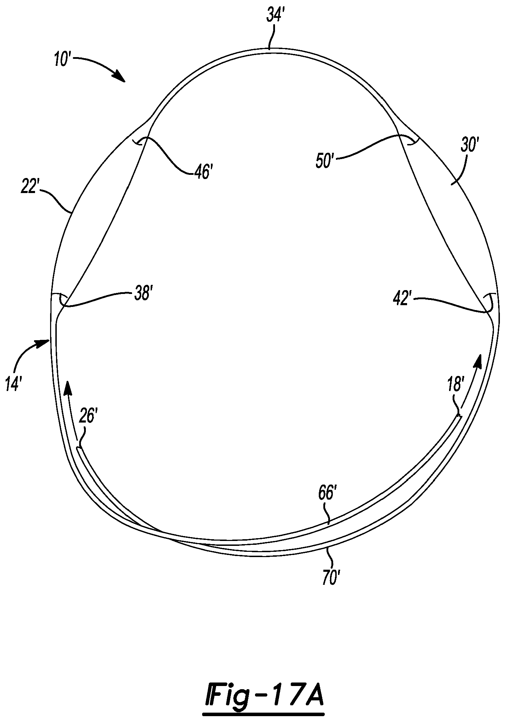

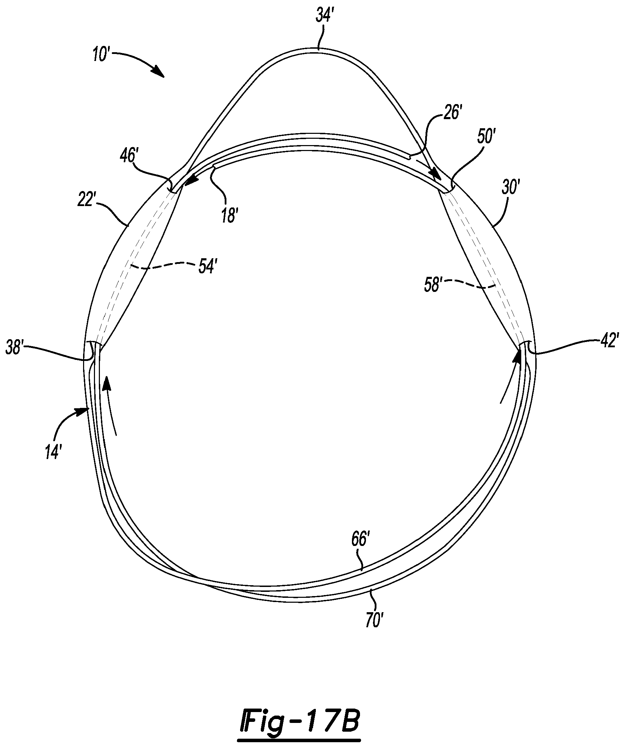

Referring to Figure 17, an adjustable flexible member construct 10' is provided according to various aspects of the present teachings. The adjustable flexible member construct 10' can be fashioned from a flexible member 14' made of any biocompatible material including, but not limited to, non-resorbable polymers, such as polyethylene or polyester, resorbable polymers, and various combinations thereof. In various aspects, the adjustable flexible member construct 10' can include a hollow material or core to allow for appropriate tensioning, as will be discussed herein. In various aspects, the adjustable flexible member construct 10' can be a suture. In such aspects, the suture can be hollow or a braided or a multiple-filament braided suture structure having a hollow core. In various aspects, the suture can be resorbable. In various aspects, the adjustable flexible member construct 10' can define a substantially tubular hollow shape.

The adjustable flexible member construct 10' can include a first end 18', a first formed passage portion 22', a second end 26', a second formed passage portion 30', and a fixed length loop portion 34' connecting the first and second passage portions 22', 30', as shown in Figure 17. In one exemplary aspect, flexible member construct 10' can include an elongated body 32' having an exterior surface and an interior surface defining an elongated passage between the first and second ends 18', 26'. The body 32' can define the first and second passage portions 22', 30' and the fixed length portion 34' therebetween. Passage portions 22', 30' can each include first apertures 38', 42' positioned proximate one end thereof, and second apertures 46', 50' positioned proximate a second opposite end thereof. The passage portions 22', 30' can be formed to have a larger width or diameter than remaining portions of flexible member 14', as shown for example in Figure 17. Alternatively, the passage portions 22', 30' can be formed initially to have the same width or diameter as the remaining portions of flexible member 14', later expanding in diameter during the construction process. In various aspects, the first and second apertures 38', 42', 46', 50' can be formed during a braiding process of flexible member 14' as loose portions between pairs of fibers defining flexible member 14', or can be formed during the construction process. Alternatively, the first and second ends can be pushed between individual fibers of the braided flexible member 14', as will be discussed herein.

To form the adjustable flexible member construct 10', first end 18' can be passed through second passage portion 30' via first and second apertures 42', 50', as generally shown in Figures 17A and 17B. In a similar manner, second end 26' can be passed through the first passage portion 22' via the first and second apertures 38', 46', as also shown in Figures 17A and 17B. Subsequently, as shown in Figure 17B with reference to Figure 17, first end 18' can be passed through the first passage portion 22' via second and first apertures 46' and 38', respectively. First end 18' can follow a path that is opposite in direction to a path followed by a portion 54' of the flexible member 14' that has already passed through first passage portion 22' while following second end 26' through first and second apertures 38' and 46'. Similarly, second end 26' can be passed through the second passage portion 30' via second and first apertures 50' and 42', respectively. First end 26' can follow a path that is opposite in direction to a path followed by a portion 58' of the flexible member 14' that has already passed through second passage portion 30' while following first end 18' through first and second apertures 42' and 50'. This results in portions 62', 64' of flexible member 14' being positioned parallel or substantially parallel to portions 54', 58' in passage portions 22', 30'. Passing the first and second ends 18', 26' though passage portions 22', 30' as discussed above forms adjustable loops 66', 70', as shown in Figure 17. The first and second ends can be passed through the same apertures in each passage portion 22', 30' or, alternatively, through separate apertures in each passage portion 22', 30'.

The adjustable flexible member construct 10' can thus provide a double adjustable loop configuration via loops 66', 70' while also providing portion 34', which can have a fixed length between the passage portions 22', 30'. As will be discussed in greater detail herein, this configuration can be used, for example, to couple an attachment member to loops 66', 70' and couple fixed length portion 34' to either the attachment member or another device. In this manner, the amount of friction developed within the first and second passage portions 22', 30' relative to and among portions 54', 58', 62' and 64' during adjustment of adjustable loops 66', 70' is reduced as compared to that which would occur if the attachment member were coupled to the passage portion when the loops are being adjusted or reduced in size under tension.

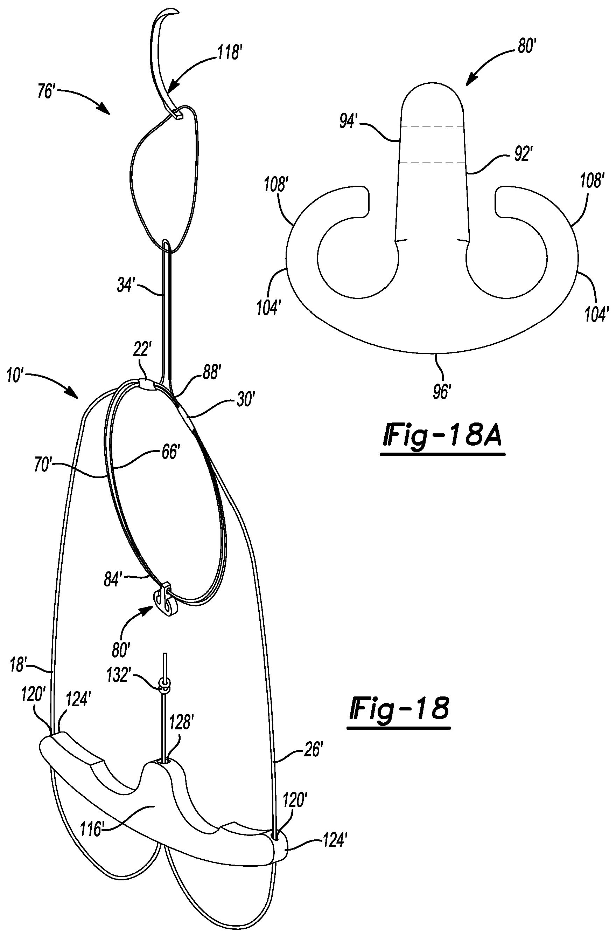

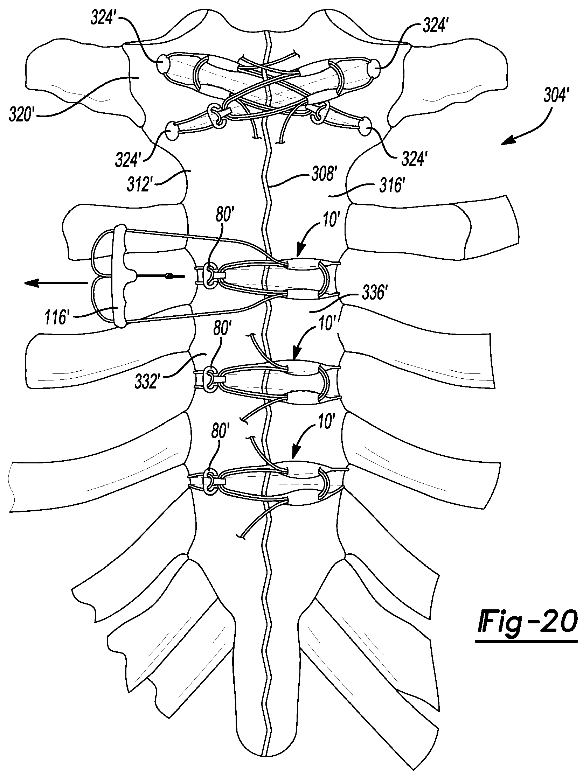

With additional reference to Figures 18 and 18A, adjustable flexible member construct 10' is shown in an exemplary assembly configuration 76 having an attachment member 80' coupled to a first side 84' of loops 66', 70' opposite a second side 88' facing fixed length portion 34'. Attachment member 80' can include a generally T-shaped configuration having a first stem portion 92' defining an aperture 94' for receipt of loops 66', 70' therein at one end, and a transversely extending cross portion 96' at a second opposite end. Transversely extending portion 96' can include opposed lateral ends 104' that include arcuate or curled portions 108', as shown in Figure 18A. In various aspects, attachment member 80' can be used to secure a flexible member loop thereto by placing the loop over first portion 92' and under arcuate portions 108', as shown for example in Figure 20.

The assembly configuration 76' can also include an optional grab member or handle 116' and a passing or needle member 118'. Handle 116' can be used to aid the surgeon in easily pulling ends 18', 26' of construct 10' to reduce the size of loops 66', 70', as will be discussed in greater detail below. Handle 116' can include a first pair of apertures 120' positioned at opposed ends 124' of handle 116', as shown in Figure 18. The first and second ends 18', 26' can be passed or routed through apertures 120' and then through a central aperture 128', where ends 18', 26' can be secured to handle 116' by various methods, including a knot 132', as also shown in Figure 18. The surgeon can use handle 116' to apply simultaneous tension to ends 18', 26', which can thereby evenly reduce or adjust loops 66', 70' to a desired size or tension.

Operation of the adjustable flexible member construct 10' will now be described in greater detail with reference to an exemplary configuration where adjustable flexible member construct 10' is wrapped around or encircles a bone, such as a sternum, and fixed loop 34' is connected to attachment member 80', as shown for example in Figure 20. It should be appreciated, however, that construct 10' can be used in various attachment configurations, other than the example discussed above, wherein tension is applied to construct 10' via fixed loop 34' and attachment member 80' in connection with reducing or adjusting the size of loops 66', 70'.

Upon applying tension to ends 18', 26', with or without handle 116', the loops 66', 70' can be reduced to a desired size and/or placed in a desired tension by causing translation of ends 18', 26' relative to passage portions 22', 30'. Tension in fixed length loop portion 34' combined with the tension in adjustable loops 66', 70' can cause the body 32' of flexible member 14' defining the passage portions 22', 30' to constrict about the portions 54', 58' and 62', 64' of flexible member 14' passed therethrough. This constriction can reduce a width or diameter of each of the passage portions 22', 30', thereby forming a mechanical interface between exterior surfaces of the passed through portions of flexible member 14' and interior surfaces of the passage portions 22', 30'. The static friction between the interior and exterior surfaces at the mechanical interface formed as a result of the constriction can prevent relative movement of portions 54', 58', and 62', 64' relative to passages 22', 30' and hence prevent relaxation of the tension in construct 10', thereby preventing an increase in the size of loops 66', 70'. Thus, adjustable flexible member construct 10' provides for "automatically" locking loops 66', 70' in a reduced length or size under tension without requiring a knot.

Flexible member construct 10' can be provided in various sizes to accommodate differently sized bones, such as sternums, in different patients. In one exemplary configuration, fixed loop portion 34' can be provided in various sizes or lengths. Flexible member construct 10' can also be provided with flexible member 14' having various diameters, such as 0.762 mm or 0.940-1.016 mm (30 thousandths of an inch or 37-40 thousandths of an inch). In one exemplary configuration, the 0.762 mm (30 thousandths) diameter flexible member 14' can be used, for example, where construct 10' is routed or passed through holes drilled in the bone so that flexible member 14' can be more easily manipulated during such routing. The larger 0.940 - 1.016 mm (37-40 thousandths) diameter flexible member 14' can be used, for example, where the construct 10' is wrapped around the sternum, as will be discussed herein. Forming the construct 10', as well as other constructs discussed herein, with a larger diameter flexible member provides more surface area of the tensioned flexible member to engage the sternum or other bone, and thus distribute the compressive load over a greater area of the bone.

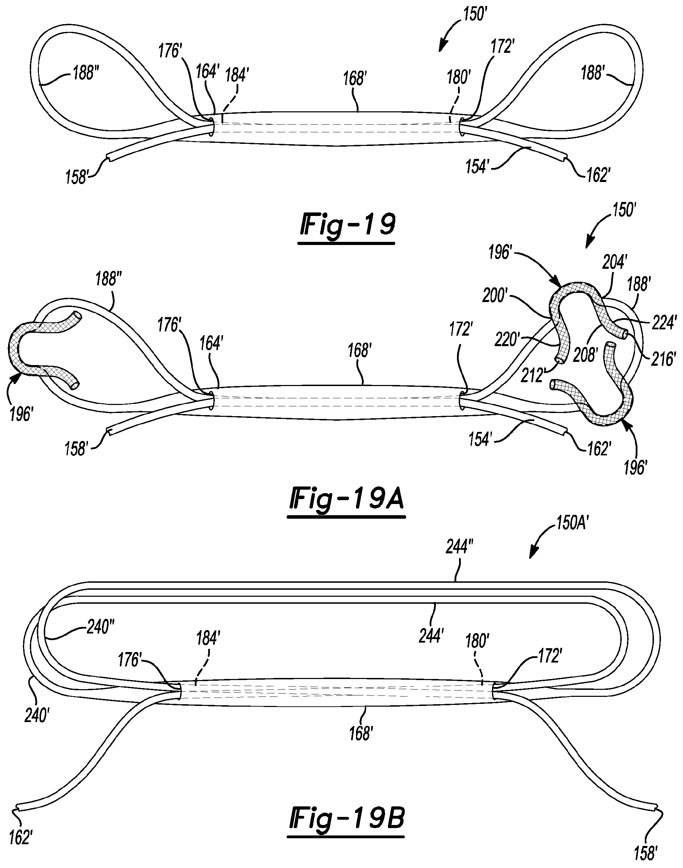

With additional reference to Figure 19, and Figure 19A, an exemplary alternative adjustable flexible member construct 150' is shown. Construct 150' can include a hollow flexible member 154' having a first end 158' and a second end 162', and can include a body 164' that defines a longitudinal passage portion 168' therein between first and second ends 158', 162', as shown in Figure 19. The passage portion 168' can define a pair of apertures 172', 176' at opposed ends thereof, similar to apertures 38', 46' discussed above. To form construct 150', the first end 158' can be passed through aperture 172' and passage portion 168' and out aperture 176' such that a portion 180' of flexible member 154' following first end 158' extends through passage portion 168'. In a similar manner, second end 162' can be passed through aperture 176' and passage portion 168' and out aperture 172' such that a portion 184' of flexible member 154' following second end 162' also extends through passage portion 168'. This configuration forms two loops 188' and 188", as shown in Figure 19. It should be appreciated that each of the first and second ends 158', 162' can alternatively be pushed through a respective space defined between adjacent individual fibers of the braided flexible member 14' such that the respective spaces defined between fibers comprise apertures 172', 176' in communication with an interior longitudinal passage.

The pulling of ends 158', 162' can cause movement of portions 180', 184' relative to passage portion 168', and the loops 188', 188" can be reduced to a desired size or placed in a desired tension. Tension in loops 188', 188" can cause the body 164' defining the passage portion 168' to be placed in tension and therefore cause passage portion 168' to constrict about portions 180", 184' passed therethrough. This constriction reduces the diameter of passage portion 168', thus forming a mechanical interface between the exterior surfaces of portions 180', 184' and an interior surface of passage portion 168'. This constriction results in static friction between the interior and exterior surfaces at the mechanical interface, causing the adjustable flexible member 154' to "automatically" lock in a reduced size or diameter configuration in which tension is maintained. Flexible member construct 150' with adjustable loops 188', 188" can be used to compress a fractured or sectioned bone, such as a sectioned sternum in a sternal closure procedure following open chest surgery, as will be discussed herein.

With additional reference to Figure 19A, adjustable flexible member construct 150' is shown having attachment members or flexible anchors 196 coupled to loops 188', 188". Each loop can include various numbers of anchors coupled thereto, including more or fewer anchors 196' than shown. Each anchor 196' can define a hollow core and can include a pair of apertures 200', 204' formed in a body 208' thereof in a similar manner as apertures 38', 46' discussed above. Flexible member 154' can pass through first aperture 204' into the hollow core and out through the second aperture 200', as shown in Figure 19A. Apertures 200', 204' can be placed inward from respective ends 212', 216' of anchors 196' so as to form tail portions 220', 224' adjacent each aperture 200', 204'. The tail portions 220', 224' can provide anchoring resistance relative to a corresponding bone or anchoring structure, as will discussed herein.

With reference to Figure 19B and continuing reference to Figures 19 and 19A, an alternative adjustable flexible member construct 150A' is shown. Construct 150A' can be formed to include a double loop configuration having two loops 240', 240" that each traverse a path from one end of passage portion 168' to the other end thereof, instead of each loop being disposed at respective opposite ends of passage portion 168' as in construct 150'. Flexible member construct 150A' can be formed by passing the first end 158' of the flexible member through aperture 176', through passage portion 168' and out aperture 172'. The second end 162 can be passed through aperture 172', through the passage portion 168 and out the aperture 176'. In various aspects, the first and second apertures 172', 176' can be formed during the braiding process as loose portions between pairs of fibers defining the flexible member 154', as discussed above. Passing ends 158', 162' through the apertures 172', 176' can form the loops 240', 240". The loops 240', 240" can define mount or summit portions 244', 244" of the adjustable flexible member construct 150A' and can be disposed generally opposite from the passage portion 168'. Flexible member construct 150A' can be used, for example, to compress a fractured or sectioned bone or to close a sectioned sternum in sternal closure procedures, as will be discussed herein.

The longitudinal and parallel placement of the first and second ends 158' and 162' of the flexible member 154' within the passage portion 168' resists the reverse relative movement of the first and second portions 180', 184' of the flexible member construct 150A' once it is tightened. The tensioning of the ends 158' and 162' can cause relative translation of the portions 180', 184' relative to passage portion 168'. Upon applying tension to the first and second ends 158' and 162', the loops 240', 240" can be reduced to a desired size or placed in a desired tension. Tension in the loops 240', 240" can cause the body of the flexible member 154' defining the passage portion 168' to be placed in tension and therefore cause passage portion 168 to constrict about the portions 180', 184' similarly to the constriction discussed above with respect to construct 150'. This constriction can cause the adjustable flexible member construct 150A' to "automatically" lock in a reduced size or smaller diameter configuration. A further discussion of the flexible member constructs 150', 150A' are provided in U.S. Patent Serial No. 11/541,506 filed on September 29, 2006 entitled "Method and Apparatus for Forming a Self-Locking Adjustable Suture Loop" assigned to Biomet Sports Medicine, LLC.

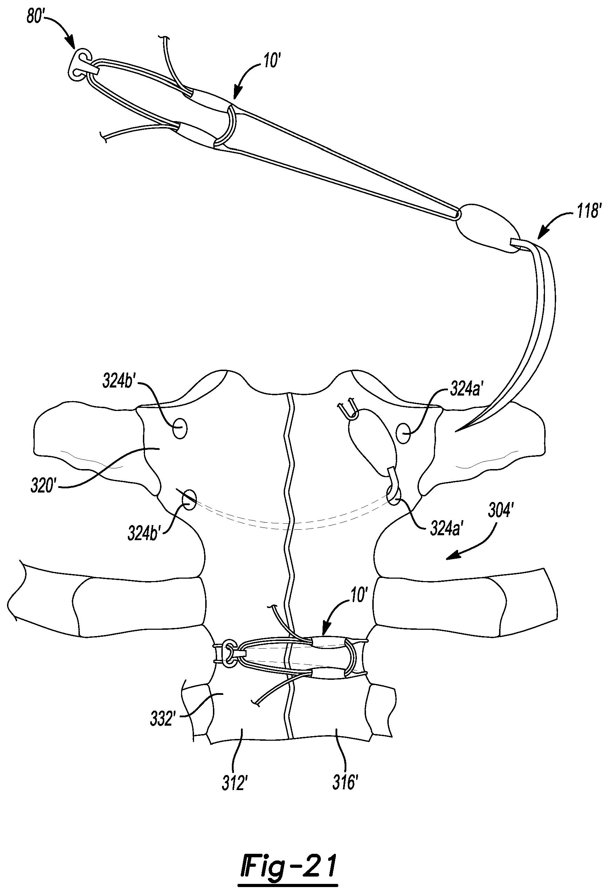

Referring now to Figures 20-32, the use of flexible member constructs 10', 150' and 150A' in various assembly configurations and exemplary sternal closure procedures will now be described. With particular reference to Figures 20 and 21, a sternum 304' is shown having a section or cut 308' separating sternal portions 312', 316', such as may be performed in connection with cardiac surgery. Flexible member constructs 10', 150', 150A' alone, or in various combinations with each other or additional fixation devices, can be used to compress and secure sternal portions 312', 316' together to assist healing, as will be discussed herein.

In Figures 20 and 21, adjustable flexible member construct 10' is shown in various configurations to compress sternal portions 312', 316' toward each other to close section 308'. In one exemplary configuration, two flexible member constructs 10' can be used in a diagonal pattern in the manubrium 320' of the sternum in connection with two pairs of diagonally opposite holes 324' formed in the manubrium 320'. While the diagonal pattern of flexible member construct 10' is shown in the manubrium 320' in Figure 20, a non-diagonal or medial-lateral configuration can alternatively be used, as generally shown in Figure 21.

To secure flexible member construct 10' to the manubrium 320', passing member 118' can be inserted through a first hole 324a' of a respective pair of holes 324a', 324b' and directed towards a corresponding second hole 324b', as shown in Figure 21. A surgeon or the like can pull the passing member through the second hole 324b' thus routing at least the fixed portion 34' through the first and second holes 324a', 324b'. Fixed portion 34' can then be secured to attachment member 80', as shown in Figure 20. Once fixed portion 34' is secured to the attachment member, first and second ends 18', 26' can be pulled or tensioned to reduce the loops 66', 70' to a desired size and to place construct 10' in a desired tension to compress and close the sectioned sternum 304'. Ends 18', 26' of construct 10' can be tensioned by pulling on the respective ends as discussed above, or with the use of the handle 116', as generally shown in Figure 20. Handle 116' can provide the surgeon with an ability to easily tension ends 18', 26' simultaneously and evenly. Handle 116' can then be removed and discarded. Handle 116' can be used to evenly tension loops 66', 70' as discussed above, or can be used to tension loops 66', 70' at different rates by manipulating an angle of handle 116' so that, for example, a first loop of loops 66', 70' can be tensioned at a faster rate than a second loop of loops 66', 70'. In this manner, the first loop can reach a desired final tension before the second loop. In one exemplary configuration, the smaller diameter flexible member can be used with construct 10' in manubrium 320' for easier manipulation through holes 324'.

Flexible member construct 10' can also be used to compress a body 332' of sternum 304', as also shown in Figures 20 and 21. For the body 332', construct 10' can be wrapped around the sternum and fixed portion 34' can be secured to attachment member 80' such that ends 18', 26' extend from an anterior side 336' of body 332', as shown in Figure 20. In the exemplary configuration shown in Figure 20, three flexible member constructs 10' are shown securing the body 332' of the sternum 304'. Nevertheless, more or fewer flexible constructs than shown can be used in the intercostal spaces between the ribs to secure the body of the sternum, as may be determined by a surgeon during a sternal closure procedure. In addition, the larger diameter flexible member construct 10' can be utilized in body area 332' of sternum 304', according to one exemplary configuration. The larger diameter flexible member can enable more tension to be applied to the bone or sternum without cutting into or damaging the bone.

The flexible member constructs 10' can be attached and tensioned or secured to the sternum 304' in various orders. For example, flexible member constructs 10' can first be attached to the manubrium 320' and then to the body 332', or vice-versa. Additionally, flexible member constructs 10' can be tensioned in various orders, such as initially tensioning each flexible member construct 10' to a snug or non-slack condition and then further tensioning each construct 10' to a final desired tension. As discussed above, constructs 10' can be tightened with or without use of handle 116'. Flexible construct 10' can automatically lock under tension, as also discussed above, after which a portion of ends 18', 26' can be trimmed and removed.

Flexible member construct 10' also can be provided with an antibiotic and/or platelet concentrate coating to resist bacterial adhesion and/or promote healing. In this regard, flexible member construct 10', as well as other constructs discussed herein, can be pre-configured with such a coating or the coating can be applied intraoperatively. Further, the surgeon can also apply the platelet coating to the sectioned area during the sternal closure procedure.

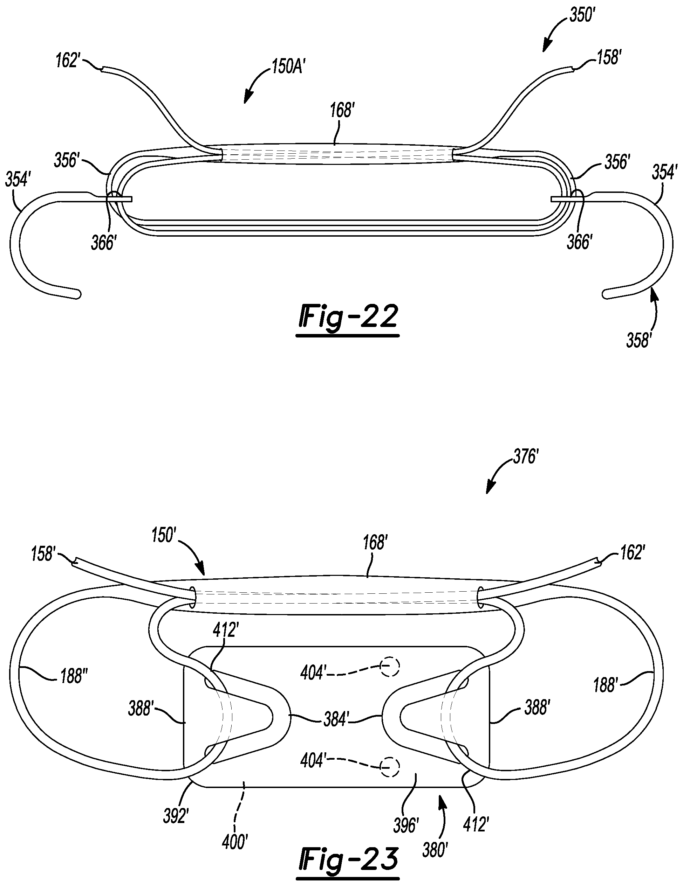

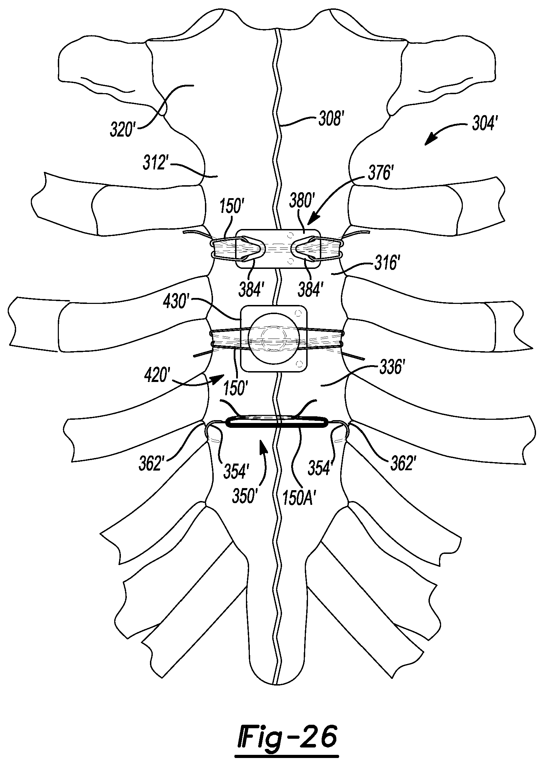

With additional reference to Figures 22 and 26, flexible member construct 150A' is shown in an assembly configuration 350' having a pair of attachment members 354' coupled to opposed sides 356' of loops 240' and 240". Attachment members 354' can include a generally arcuate shape 358' and, in the exemplary configuration shown in Figure 22, a generally semicircular shape or U-shape. The shape 358' of attachment members 354' can be used to secure the attachment members 354' to medial and lateral sides 362' of sternum 304', as generally shown in Figure 26. Attachment members 354' can include an aperture 366' for receiving loops 240', 240" therethrough, as shown in Figure 22. In an exemplary configuration, flexible member construct 150A' can be formed integrally with attachment members 354' for use in a sternal closure or other fracture reduction procedure. In this manner, attachment members 354' can be preformed and coupled to loops 240', 240" to form assembly configuration 350', which can be provided in the assembly configuration for use in the sternal closure procedure.

With particular reference to Figure 26, construct 150A' in the assembly configuration 350' can be used to compress the sternum 304' by securing attachment members 354' to the sides 362' of sternal portions 312', 316' and then applying tension to ends 158', 162' of construct 150A'. The adjustable loops of construct 150A' can then be reduced to the desired size and placed in the desired tension to compress sternum 304' about section 308'. Flexible member construct 150A' can automatically lock under tension to maintain the reduced size of loops 240', 240", as discussed above. It should be appreciated that while attachment members 354' are described above in connection with flexible member construct 150A', the attachment members 354' can also be used with alternative flexible member constructs, such as construct 150'.

Turning now to Figure 23 and with reference to Figure 26, flexible member construct 150' is shown in an assembly configuration 376 operatively associated with an attachment member or frame 380'. Frame 380' can be used to facilitate securing flexible member construct 150' around a fractured bone or the sectioned sternum 304' to compress the fracture or section and affect healing. Frame 380' can include a pair of attachment portions 384' at opposed ends 388' of the frame. In the exemplary configuration shown, frame 380' can include a generally rectangular plate 392' and the attachment portions 384' can be in the form of V-shaped apertures extending through plate 392' from a top surface 396' to a bottom surface 400', as shown in Figure 23. Bottom surface 400' can optionally include a pair of fixation members 404' to prevent movement of frame 380' relative to the sternum 304' upon placement thereon. Fixation members 404' can include spikes, posts, screws, adhesive or the like that are coupled to or pass through or extend from the bottom surface 400'.

With additional reference to Figure 26, flexible member construct 150' in the assembly configuration 376' is shown with the bottom surface of frame 380' positioned on the anterior side 336' of sternum 304'. Flexible member construct 150' can then be wrapped around sternum 304' and opposed ends 412' of loops 188', 188" can be secured to frame 380' via attachment portions 384'. In this configuration, passage portion 168' can be positioned on a posterior side of sternum 304', as generally shown in Figure 26. Ends 158', 162' of construct 150' can then be tensioned to reduce the loops 188', 188" to the desired size and tension to compress and close section 308' and assist healing of sternum 304'. Flexible member construct 150' can automatically lock and maintain the reduced size of loops 188', 188" under tension, as discussed above. It should be appreciated that frame 80' can also be used with flexible member construct 10'.

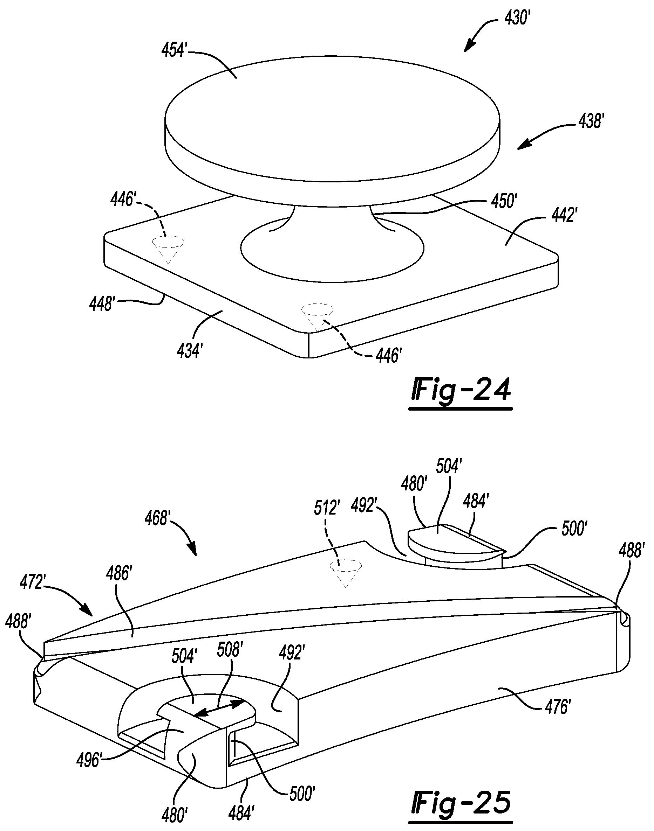

Referring now to Figures 24 and 26, flexible member construct 150' is shown in an assembly configuration 420' operatively associated with an attachment member or frame 430'. Frame 430' can include a base 434', a post 438' extending from an upper surface 442' of base 434', and at least one optional fixation member 446' extending from a lower surface 448' of base 434'. Fixation member 446' can include spikes, posts, screws, adhesive or the like that are coupled to or extend from lower surface 448'. Post 438' can include a reduced diameter neck portion 450' coupled to the base and a larger diameter or head portion 454' coupled to the neck portion 450' and configured to retain loops 188', 188" of construct 150', as discussed below. Frame 430' can be placed on sternum 304' with lower surface 448' engaging the anterior side 336' of sternum 304', as shown in Figure 26. Flexible member construct 150' can be wrapped around sternum 304' in one of the intercostal spaces and each loop 188', 188" can be secured to the frame 380' via post 438', as also shown in Figure 26.

In this exemplary configuration, passage portion 168' can be positioned on the posterior side of sternum 304'. Once frame 430' is positioned and construct 150' is wrapped around the sternum and secured to post 438', ends 158' and 162' extending from the posterior side of sternum 304' can be tensioned. Applying tension to ends 158', 162' can reduce loops 188', 188" to a desired size and tension to compress sectioned sternal portions 312', 316' together to assist healing at section 308', as generally shown in Figure 26. Flexible construct 150' can automatically lock loops 188', 188" under tension to maintain the reduced size of loops 188', 188" and compression of sternal portions 312', 316' together, as discussed above.

It should be appreciated that while reference to Figure 26 has been made with respect to the assembly configurations 350', 376' and 420', these assembly configurations have been combined in one figure for illustration purposes only and need not be used together. In this regard, an exemplary sternal closure procedure could utilize only one of the assemblies shown in the intercostal spaces, or combinations thereof, as may be desired by a particular surgeon performing a sternal closure procedure. It should also be appreciated that assemblies 350', 376' and 420' could be used individually or in various combinations with flexible member constructs 10' secured to the manubrium 320', as discussed above with reference to Figure 20.

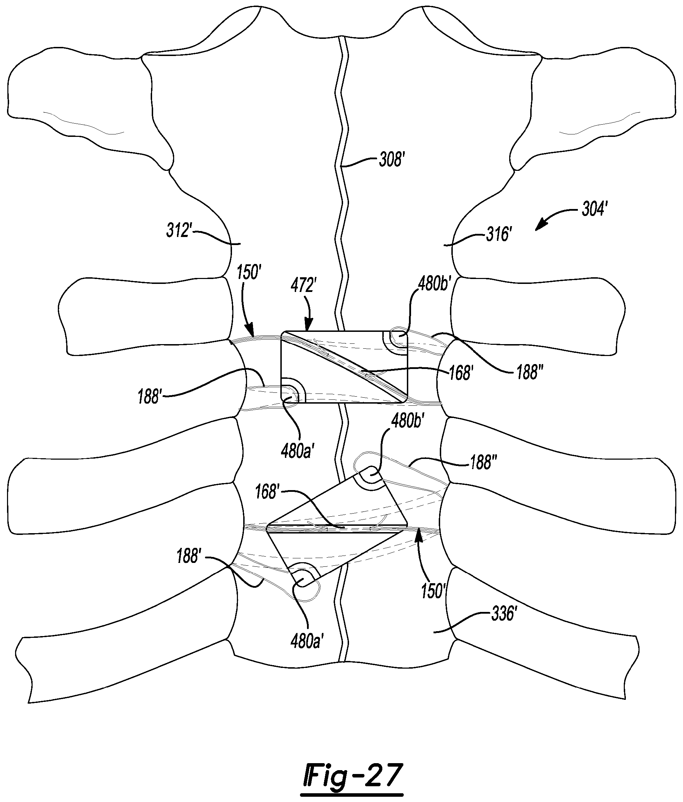

Referring now to Figures 25 and 27, adjustable flexible member construct 150' is shown in an assembly configuration 468' operatively associated with a frame 472'. As shown in Figure 25, frame 472' can include a generally rectangular body 476' with attachment portions 480' positioned at one pair of diagonally opposed corners 484' and a groove or channel 486' extending diagonally across a top surface of frame 472' from a second pair of opposed corners 488'. While frame 472' is shown having rectangular body 476', it should be appreciated that frame 472' can be configured in other shapes, such as various polygonal shapes for use in coupling frame 472' to flexible member construct 150', as will be described below. Attachment portions 480' can each include a recess 492' at least partially surrounding a post 496'. Post 496' can include a neck portion 500' and a cap or head portion 504 having a width dimension 508' greater than a corresponding width of neck portion 500' such that at least a portion of head portion 504' overhangs neck portion 500', as shown in Figure 25. At least one optional fixation member 512' can extend from a bottom surface 516' of frame 472'.

With particular reference to Figure 27, frame 472' can be positioned in various configurations relative to sternum 304', as illustrated by the two exemplary configurations shown in Figure 27. Frame 472' can be positioned on sternum 304' such that the bottom surface 516' engages the anterior side 336' of sternum 304'. Flexible member construct 150' can be wrapped around sternum 304' within an intercostal space thereof and loops 188', 188" can be coupled to respective attachment portions 480'. More specifically, flexible construct 150' can be placed in channel 486' such that passage portion 168' is positioned within channel 486', as shown in Figure 27. Positioning construct 150' in channel 486' can provide a low profile closure arrangement that can be more conformable or provide less discomfort to a recipient patient. Loop portion 188' can then be wrapped around the posterior side of sternum 304' in one direction and coupled to attachment portion 480A' of the pair of attachment portions 480'. Similarly, loop portion 188" can be wrapped around the posterior side of sternum 304' in an opposite direction of loop 188' and then be coupled to attachment portion 480B'. Tension can then be applied to ends 158', 162' to reduce the size of loops 188', 188" to compress sternal portions 312', 316' together to assist healing of sectioned sternum 304' at section 308'. Flexible member construct 150' can automatically lock loops 188', 188" at the desired reduced size under tension, as discussed above. In addition, placing frame 472' over the section can also stabilize the sternum to align sternal portions 312', 316' to be co-planar.

Flexible member construct 150' in the assembly configuration 468' can be used alone or in various combinations with flexible member constructs 10' and 150A' and/or assembly configurations 350', 376' and 420' discussed above. For example, flexible member constructs 10' can be used in the manubrium 320' as shown in Figure 20 and assembly 468' can be used alone or in various combinations with assemblies 350', 376' and 420' in the body 332' to compress sternal portions 312', 316', as discussed above.

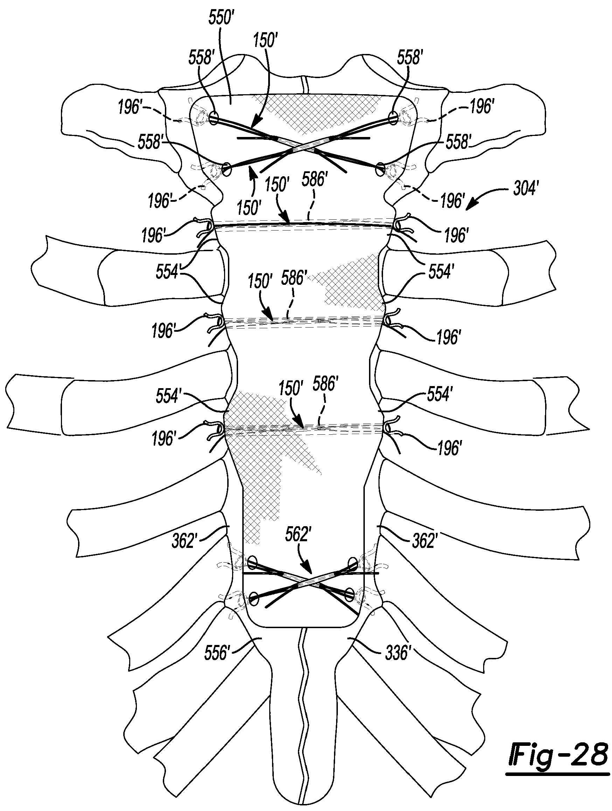

Referring now to Figure 28, adjustable flexible member construct 150' is shown operatively associated with anchors 196' and an orthopedic mesh 550' for use in a sternal closure procedure. More particularly, orthopedic mesh 550' can be positioned on the anterior side 336' of sternum 304' such that portions 554' extend around the lateral sides 362' in the intercostal spaces, as shown in Figure 28. The orthopedic mesh 550' can be, for example, a product sold by Biomet Sports Medicine, LLC under the name SportMesh™. With the orthopedic mesh 550' positioned on sternum 304' as discussed above, construct 150' with anchors 196' can be used in various configurations to compress the sectioned sternum 304' at section 308', as generally shown in Figure 28.

The orthopedic mesh 550' can be coated with the platelet concentrate discussed above, and/or antibiotics, bone growth agents, etc. to aid in soft tissue healing. The mesh 550' can provide a barrier between the flexible member constructs and the bone to aid in transferring load from the flexible member construct to the mesh 550', which can decrease the pressure applied to the bone by the tensioned flexible member construct. The mesh 550' can be particularly useful, for example, in patients with soft bone tissue. It should also be appreciated that load distribution in the intercostal spaces can be provided by the portions 554' that extend around the medial and lateral sides. Moreover, the orthopedic mesh can aid in the retention of anchors 196', particularly where the bone tissue may be soft.

In one exemplary configuration, four holes 558' can be formed through the mesh 550' and the manubrium 320'. Flexible anchors 196' associated with two flexible member constructs 150' can be inserted through respective diagonal pairs of holes 558' through the manubrium, as shown in Figure 28. The constructs 150' can be in diagonal overlapping pattern and be disposed primarily on top of the orthopedic mesh 550'. Upon tensioning the free ends 158', 162' of each construct 150', the tail portions 220', 224' of anchors 196' can engage the posterior manubrium adjacent holes 558' and provide anchoring resistance to retain the anchors 196' outside of holes 558' on the posterior side of the sternum 304'. The loops 188', 188" subsequently can be reduced to the desired size or tension to compress sternal portions 312', 316' and assist closure and healing of the sectioned sternum. The orthopedic mesh 550' can work to distribute the load placed on the anterior side 336' of the sternum by the constructs 150' under tension. A similar configuration 562' can be used at a lower portion 566' of the sternum 304' adjacent the Xiphoid process, as also shown in Figure 28. It should be appreciated that configuration 562', as well as the configuration discussed immediately above with respect to the manubrium, can alternatively be in a parallel transverse pattern as opposed to the illustrated diagonal patterns.

Continuing with Figure 28, adjustable flexible construct 150' having a pair of anchors 196' attached to respective loops 188', 188" can be inserted through transverse bores 586' formed in sternum 304'. In particular, the constructs 150' can be positioned in bores 586' such that the passage portions 168' are each aligned in a respective bore 586', as shown in Figure 28. The constructs 150' can be pierced or routed through the portions 554' of orthopedic mesh 550' that extend around the lateral sides of sternum 304' so as to provide additional anchoring resistance and load distribution for flexible anchors 196', as shown in Figure 28. Ends 158', 162' can be tensioned to compress sternal portions 312', 316', as discussed herein. It should be appreciated that while orthopedic mesh 550' is shown in Figure 28 with reference to construct 150' and flexible anchors 196', orthopedic mesh 550' can be used in various other sternal closure configurations disclosed herein, for example, to distribute a load applied by the various disclosed flexible member constructs relative to the sternum 304'.

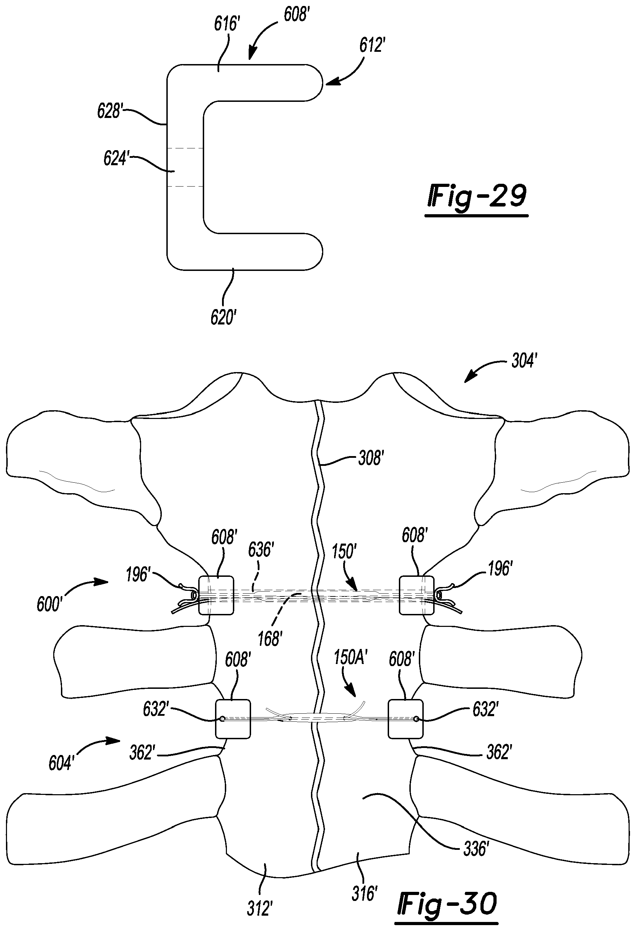

Referring now to Figures 29 and 30, adjustable flexible member construct 150' is shown in exemplary assembly configurations 600' and 604' operatively associated with attachment members 608'. Each attachment member 608' can include a body 612' having a substantially U-shaped configuration and can be sized for positioning about the lateral sides 362' of sternum 304' such that top and bottom portions 616', 620' extend about the respective anterior and posterior sides of the sternum, as shown in Figure 30. In one exemplary configuration, attachment member 608' can include an aperture 624' positioned within a side portion 628' connecting the top and bottom portions 616', 620'. In another configuration, attachment member 608' can include an aperture 632' in the top portion 616', as shown in Figure 30. Aperture 632' can be in lieu of or in addition to aperture 624'.

With reference to assembly configuration 600', flexible member construct 150' with anchors 196' can be positioned through transverse bore 636' in sternum 304' such that passage portion 168' is positioned within the bore. Each respective loop 188', 188" with anchors 196' can be passed through aperture 62'4 in attachment member 608' such that the anchors 196' are on a first side of portion 628' opposite a second side adjacent the sternum 304'. Ends 158', 162' can then be tensioned thereby reducing a size of loops 188', 188" so as to draw attachment members 608' against the lateral sides of sternum 304' and compress sternal portions 312', 316' together. Flexible member construct 150' can automatically lock the loops in the reduced diameter configuration under tension, as described herein. Attachment members 608' can facilitate distributing a compression load applied to the sternum by the tensioned construct 150', which can enable more tension to be applied.

With continuing reference to Figure 30, assembly configuration 604' can include attachment members 608' integrally formed or pre-assembled with flexible member construct 150A' such that loops 240', 240" are coupled to apertures 632'. In this configuration, attachment members 608' can be positioned against the respective lateral sides of sternum 304', similar to assembly configuration 600' discussed above. Flexible member construct 150A' can be positioned relative to the anterior side 336' of sternum 304' such that it does not wrap around or extend through sternum 304'. It should be appreciated that assembly configurations 600', 604' can be used alone or with various other flexible member construct and assembly configurations disclosed herein to compress sternal portions 312', 316' to assist healing of sectioned sternum 304'.

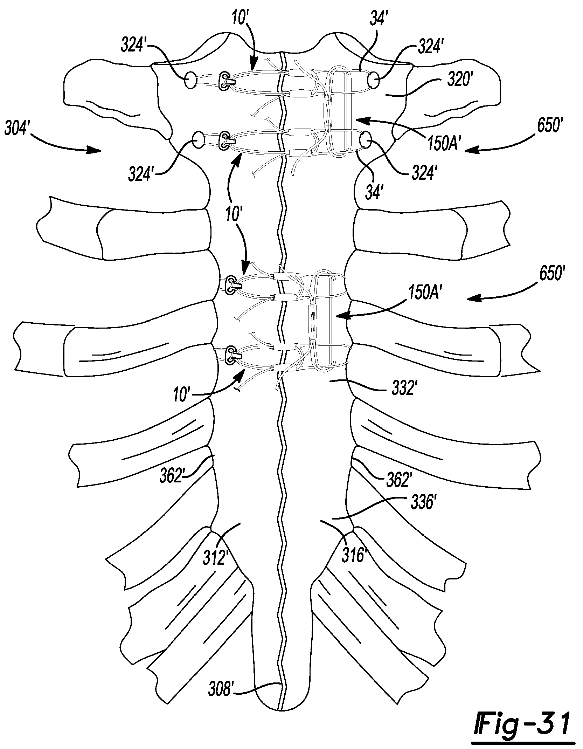

Turning now to Figure 31, an alternative configuration 650' for compression of sternum 304' in a sternal closure procedure is provided. Configuration 650' can include two flexible member constructs 10' in a transverse orientation in the manubrium 320', as generally discussed above with reference to Figures 20 and 21. Configuration 650' can also include flexible member construct 150A' coupled around the two constructs 10' before fixed portion 34' of each construct 10' is coupled to the respective attachment member 80', as shown in Figure 31. Construct 150A' can be positioned generally in a superior-inferior orientation perpendicular to the transverse orientation of constructs 10'. Construct 150A' can be tensioned after tensioning constructs 10' to draw any remaining tension from the system. In this configuration, constructs 10' can provide cross-tensioning generally perpendicular to section 308' and construct 150A' can provide tensioning generally parallel to section 308'. Configuration 650' can also be utilized to compress the body 332' of sternum 304', where constructs 10' are wrapped around the sternum 304' instead of through holes 324', as also shown in Figure 31.

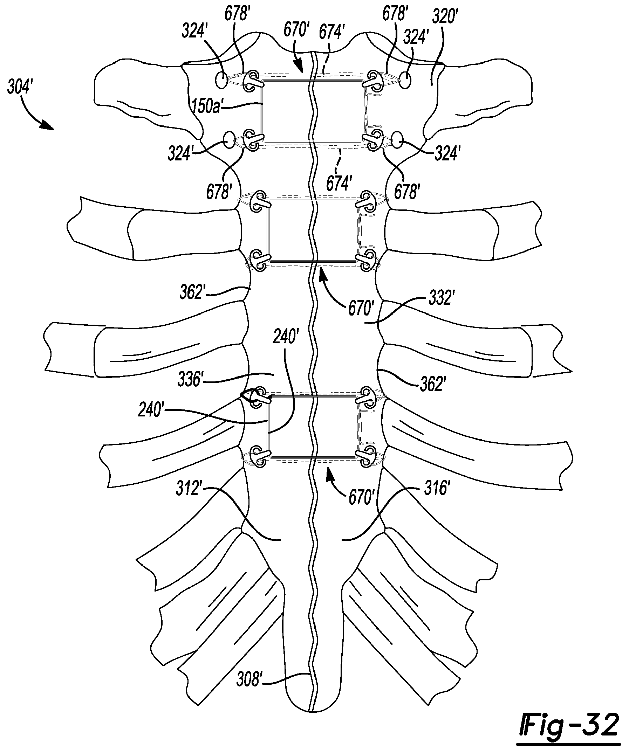

Referring now to Figure 32, another alternative configuration 670' for compression of sternum 304' in a sternal closure procedure is provided. Configuration 670' can include two separate continuous suture or flexible member loops 674' having a fixed length. Alternatively, a flexible member construct, such as construct 150', can be used in place of fixed loops 674' to provide additional adjustment and tensioning capability. Flexible member construct 150A' can be provided with four attachment members 80' integrally coupled to loops 240', 240". In the manubrium area, loops 674' can be routed or passed along the posterior side of sternum 304' in a transverse orientation such that opposed ends 678' of loops 674' extend through a respective pair of holes 324', as shown in Figure 32. The opposed ends 678' of each of loops 674' can be coupled to a respective two of the four attachment members 80'. The ends 158', 162' of flexible member construct 150A' can then be tensioned to compress sternal portions 312', 316', as discussed herein. Additional configurations 670' can be used to compress body 332' of sternum 304' where the fixed loops are wrapped around the sides of sternum 304' as opposed to being passed through holes 324', as also shown in Figure 16. It should be appreciated that configurations 650' and 670' can be used alone or with various combinations of the flexible member constructs and assembly configurations discussed herein.

The description of the invention is merely exemplary in nature and, thus, variations that do not depart from the gist of the invention are intended to be within the scope of the invention. For example, any of the above mentioned surgical procedures is applicable to the repair of other body portions. For example, the procedures can be equally applied to orthopedic repair of wrists, fingers, legs, ankles, and other bones and also to non-orthopedic repairs. Such variations are not to be regarded as a departure from the scope of the invention.

## Claims

1. An apparatus for securing a fractured or sectioned sternum (304') in a patient's body, comprising:
an attachment member (80') having a first portion (92') and a second portion (96'); and
an adjustable flexible member construct (150') having a body (164') extending from a first end (158') to a second end (162') and defining at least one passage portion (168'), the first and second ends (158', 162') being passed into and through the at least one passage portion (168') via at least first and second openings (172', 176') associated with the at least one passage portion (168') of the adjustable flexible member construct (150') to form a pair of loops (188');
wherein the attachment member (80') is configured to be coupled to at least one of the formed pair of loops (188');
**characterised in that**
the second portion (96') is positioned substantially perpendicular to the first portion (92') to form a generally T-shaped configuration of the attachment member (80'), the first portion (92') forming a flexible member holder having an aperture (94') formed in the first portion (92') proximate an end opposite the second portion (96').

2. The apparatus of claim 1, wherein the at least one passage portion (168') includes first and second passage portions each having at least the first and second openings (172', 176') and spaced apart from each other by a connecting portion of the adjustable flexible member construct (150'), the first end (158') being passed into and through the second passage portion and then into and through the first passage portion to form a first adjustable loop of the pair of loops (188'), the second end being passed though the first passage portion and then through the second passage portion to form a second adjustable loop of the pair of loops (188'), the first and second loops having a first side adjacent the portion and a second side opposite the connecting portion.

3. The apparatus of claim 2, wherein the first and second ends (158', 162') are passed through both the first passage portion in opposite directions and the second passage portion in opposite directions.

4. The apparatus of claim 3, wherein the first end (158') is passed through the second passage portion from the first opening (172') to the second opening (176') thereof and is passed through the first passage portion from second opening (176') to the first opening thereof; and
wherein the second end (162') is passed through the first passage portion from the first opening (172') to the second opening (176') thereof and is passed through the second passage portion from second opening (176') to the first opening (172') thereof.

5. The apparatus of claim 3, wherein the first and second ends (158', 162') are moveable relative to the first and second passage portions such that the first and second loops can be adjusted in size relative to the first and second passage portions.

6. The apparatus of claim 3, wherein the connecting portion is fixed in length between the first and second passage portions.

7. The apparatus of claim 1, wherein the adjustable flexible member construct(150') comprises a suture formed of a braided or woven structure.

8. The apparatus of claim 7, wherein the first and second loops are passed through the aperture (94') of the attachment member (80') to slidably couple the attachment member (80') thereto.

9. The apparatus of claim 2, further comprising a passing member (118') detachably coupled to the connecting portion.

10. The apparatus of claim 2, further comprising a tensioning member removably coupled to the first and second ends (158', 162') of the adjustable flexible member construct (150').

## Patentansprüche

1. Vorrichtung zum Fixieren eines gebrochenen oder geteilten Brustbeins (304') im Körper eines Patienten, umfassend:
ein Befestigungselement (80') mit einem ersten Abschnitt (92') und einem zweiten Abschnitt (96');
und
ein Gebilde (150') aus verstellbaren flexiblen Elementen, welches einen Körper (164') hat, der sich von einem ersten Ende (158') zu einem zweiten Ende (162') erstreckt, und welches mindestens einen Durchgangsabschnitt (168') definiert, wobei das erste und zweite Ende (158', 162') in den mindestens einen Durchgangsabschnitt (168') über mindestens die erste und zweite Öffnung (172', 176'), die zu dem mindestens einen Durchgangsabschnitt (168') des Gebildes (150') aus verstellbaren flexiblen Elementen gehören, hinein- und hindurchgeführt werden, um ein Schlaufenpaar zu bilden (188');
wobei das Befestigungselement (80') konfiguriert ist, um an mindestens eines der gebildeten Schlaufenpaare gekoppelt zu werden (188');
**dadurch gekennzeichnet, dass**
der zweite Abschnitt (96') im Wesentlichen senkrecht zum ersten Abschnitt (92') angeordnet ist, um eine allgemein T-förmige Konfiguration des Befestigungselements (80') zu bilden, wobei der erste Abschnitt (92') einen flexiblen Elementhalter bildet, welcher eine Öffnung (94') hat, die im ersten Abschnitt (92') nahe eines Endes gegenüber des zweiten Abschnitts (96') gebildet wird.

2. Vorrichtung nach Anspruch 1, wobei der mindestens eine Durchgangsabschnitt (168') einen ersten und zweiten Durchgangsabschnitt umfasst, die jeweils mindestens die erste und zweite Öffnung (172', 176') haben und durch einen verbindenden Abschnitt des Gebildes (150') aus verstellbaren flexiblen Elementen voneinander beabstandet sind, wobei das erste Ende (158') in den zweiten Durchgangsabschnitt hinein- und durch ihn hindurch- und dann in den ersten Durchgangsabschnitt hinein- und durch ihn hindurchgeführt wird, um eine erste verstellbare Schlaufe des Schlaufenpaars (188') zu bilden, wobei das zweite Ende durch den ersten Durchgangsabschnitt und dann durch den zweiten Durchgangsabschnitt geführt wird, um eine zweite verstellbare Schlaufe des Schlaufenpaars (188') zu bilden, wobei die erste und zweite Schlaufe eine erste Seite, die neben dem Abschnitt liegt, und eine zweite Seite, die gegenüber dem verbindenden Abschnitt liegt, haben.

3. Vorrichtung nach Anspruch 2, wobei das erste und zweite Ende (158', 162') sowohl in entgegengesetzter Richtung durch den ersten Durchgangsabschnitt als auch in entgegengesetzter Richtung durch den zweiten Durchgangsabschnitt geführt werden.

4. Vorrichtung nach Anspruch 3, wobei das erste Ende (158') von der ersten Öffnung (172') durch den zweiten Durchgangsabschnitt zur zweiten Öffnung (176') geführt wird und von der zweiten Öffnung (176') durch den ersten Durchgangsabschnitt zur ersten Öffnung geführt; und
wobei das zweite Ende (162') von der ersten Öffnung (172') durch den ersten Durchgangsabschnitt zur zweiten Öffnung (176') hindurchgeführt und von der zweiten Öffnung (176') durch den zweiten Durchgangsabschnitt zur ersten Öffnung (172') hindurchgeführt wird.

5. Vorrichtung nach Anspruch 3, wobei das erste und zweite Ende (158', 162') relativ zum ersten und zweiten Durchgangsabschnitt beweglich sind, sodass die erste und zweite Schlaufe in der Größe relativ zum ersten und zweiten Durchgangsabschnitt angepasst werden können.

6. Vorrichtung nach Anspruch 3, wobei der verbindende Abschnitt in der Länge zwischen dem ersten und dem zweiten Durchgangsabschnitt befestigt ist.

7. Vorrichtung nach Anspruch 1, wobei das Gebilde (150') aus verstellbaren flexiblen Elementen einen Faden umfasst, welcher aus einer geflochtenen oder gewobenen Struktur gebildet wird.

8. Vorrichtung nach Anspruch 7, wobei die erste und zweite Schlaufe durch die Öffnung (94') des Befestigungselements (80') hindurchgeführt werden, um das Befestigungselement (80') gleitend daran zu koppeln.

9. Vorrichtung nach Anspruch 2, ferner umfassend ein hindurchführendes Element (118'), das abnehmbar mit dem verbindenden Abschnitt gekoppelt ist.

10. Vorrichtung nach Anspruch 2, ferner umfassend ein Spannelement, dass abnehmbar an das erste und zweite Ende (158', 162') des Gebildes (150') aus verstellbaren flexiblen Elementen gekoppelt ist.

## Revendications

1. Un appareil pour la fixation d'un sternum fracturé ou découpé (304') dans le corps d'un patient, comprenant :
un élément d'attache (80') comprenant une première partie (92') et une deuxième partie (96') ;
et
une structure à élément flexible ajustable (150') possédant un corps (164') s'étendant d'une première extrémité (158') à une deuxième extrémité (162'), et définissant au moins une partie de passage (168'), les première et deuxième extrémités (158', 162') étant introduites dans ladite au moins une partie de passage (168'), qu'elles traversent, à travers au moins des premières et deuxièmes ouvertures (172', 176') associées avec ladite au moins une partie de passage (168') de la structure à élément flexible ajustable (150'), en formant une paire de boucles (188') ;
l'élément d'attache (80') étant configuré pour être accouplé avec au moins une de la paire de boucles formée (188') ;
**caractérisé en ce que**
la deuxième partie (96') est positionnée de façon substantiellement perpendiculaire à la première partie (92') en formant une configuration à forme générale en T de l'élément d'attache (80'), la première partie (92') formant un support d'élément flexible possédant une ouverture (94') formée dans la première partie (92') à proximité d'une extrémité en face de la deuxième partie (96').

2. L'appareil selon la revendication 1, l'au moins une partie de passage (168') comprenant des première et deuxième parties de passage munies chacune d'au moins les premières et deuxièmes ouvertures (172', 176'), et espacées l'une de l'autre par une partie de raccordement de la structure à élément flexible ajustable (150'), la première extrémité (158') étant introduite dans la deuxième partie de passage, qu'elle traverse, puis dans la première partie de passage, qu'elle traverse, en formant une première boucle ajustable de la paire de boucles (188'), la deuxième extrémité traversant la première partie de passage puis la deuxième partie de passage, en formant une deuxième boucle ajustable de la paire de boucles (188'), les première et deuxième boucles possédant un premier côté adjacent à la partie, et un deuxième côté faisant face à la partie de raccordement.

3. L'appareil selon la revendication 2, comprenant le passage des première et deuxième extrémités (158', 162') à la fois à travers la première partie de passage, dans des directions opposées, et la deuxième partie de passage, dans des directions opposées.

4. L'appareil selon la revendication 3, comportant le passage de la première extrémité (158') dans la deuxième partie de passage de sa première ouverture (172') à sa deuxième ouverture (176'), et le passage à travers la première partie de passage de la deuxième ouverture (176') à sa première ouverture ; et
la deuxième extrémité (162') traversant la première partie de passage de sa première ouverture (172') à sa deuxième ouverture (176'), et traversant la deuxième partie de passage de sa deuxième ouverture (176') à sa première ouverture (172').

5. L'appareil selon la revendication 3, les première et deuxième extrémités (158', 162') pouvant être déplacées relativement aux première et deuxième parties de passage, de façon à pouvoir ajuster la taille des première et deuxième boucles en fonction des première et deuxième parties de passage.

6. L'appareil selon la revendication 3, la longueur de la partie de raccordement étant fixe entre les première et deuxième parties de passage.

7. L'appareil selon la revendication 1, structure à élément flexible ajustable (150') comprenant une suture composée d'une structure tressée ou tissée.

8. L'appareil selon la revendication 7,
les première et deuxième boucles passant par l'ouverture (94') de l'élément d'attache (80') pour leur accouplement par coulissement avec celles-ci.

9. L'appareil selon la revendication 2, comprenant en outre un élément de passage (118') accouplé de façon détachable à la partie de raccordement.

10. L'appareil selon la revendication 2, comprenant en outre un élément de tension accouplé de façon détachable aux première et deuxième extrémités (158', 162') de la structure à élément flexible ajustable (150').
